# EUROPEAN PATENT SPECIFICATION

(11) **EP 3 475 449 B1**
(45) Date of publication and mention of the grant of the patent: **17.08.2022**
(21) Application number: 17816242.6
(22) Date of filing: 22.06.2017
(51) Int. Cl.: C12Q 1/68, G01N 33/50, G01N 33/574, C12Q 1/6851, G16B 40/00

(54) **USES OF A CELL-FREE NUCLEIC ACID STANDARDS**
VERWENDUNGEN VON ZELLFREIE NUKLEINSÄURESTANDARDS
UTILISATIONS DES ÉTALONS D'ACIDE NUCLÉIQUE ACELLULAIRE

(30) Priority: 23.06.2016 US 201662353779 P
(43) Date of publication of application: 01.05.2019
(73) Proprietor: Accuragen Holdings Limited, George Town, KY1-1106 Grand Cayman (KY)
(72) Inventor: WENG, Li, Fremont, CA 94555 (US); LIN, Chia-hui, Redwood City, CA 94065 (US); ZHAO, Grace Qizhi, Palo Alto, CA 94306 (US); LIN, Shengrong, Fremont, CA 94555 (US)
(74) Representative: Mewburn Ellis LLP
(86) International application number: PCT/US2017/038844
(87) International publication number: WO 2017/223366

(56) References cited:
- WO-A1-2014/014498
- WO-A1-2015/089333
- US-A1- 2013 224 740
- US-A1- 2013 224 740
- DEVONSHIRE, AS ET AL.: 'Towards Standardisation of Cell -free DNA Measurement in Plasma: Controls for Extraction Efficiency, Fragment Size Bias and Quantification' ANALYTICAL AND BIOANALYTICAL CHEMISTRY vol. 406, no. 26, pages 6499 - 6512, XP035401224
- BRIETBACH, S ET AL.: 'Direct Quantification of Cell -Free, Circulating DNA from Unpurified Plasma' PLOS ONE vol. 9, no. 3, 03 March 2014, pages 1 - 11, XP055448112
- 'Creating Standard Curves with Genomic DNA or Plasmid DNA Templates for Use in Quantitative PCR. 2003' APPLIED BIOSYSTEMS, [Online] 17 October 2017, XP055448117 Retrieved from the Internet: <URL:http://www6.appliedbiosystems.com/supp ort/tutorials/pdf/quant_pcr.pdf>

## Description

### BACKGROUND OF THE INVENTION

The presence of cfDNA in plasma was first described in 1948. Recent studies have shown that the release of some cfDNA into the blood is related to apoptosis of cancer cells. It has been found that the most abundant cfDNA fragments in cancer patients were approximately 180 base pairs (bp) in size, accompanied by larger DNA fragments with size in multiples of 180 bp, which is reminiscent of the ladder pattern of nucleosomal DNA fragments shown by apoptotic cells. The cleavage of DNA into nucleosomal DNA fragments during apoptosis is mediated by Caspase-activated DNase (CAD, also known as DFF40). CAD cleaves the linker region between nucleosomes and leaves 5'-phosphate and 3'-hydroxyl groups.

Rapid development of cfDNA assay technologies, such as high-throughput next generation sequencing (NGS), qPCR or digital PCR, enables the profiling cfDNA samples. However, performance evaluation and comparison between different assays can be challenging due to sample variability and technology bias.

### SUMMARY OF THE INVENTION

cfDNA reference standards that closely resemble human cfDNA extracted from human plasma are important for evaluating and validating cfDNA assays across different platforms and labs. The currently available "artificial cfDNA" generated by mechanical shearing usually contains a heterogeneous mixture of polynucleotides having blunt, 3'- and/or 5'-overhanging ends that may lack 5'-phosphate and 3'-hydroxyl groups, while MNase digestion yields 5'-hydroxyl and 3'-phosphate nucleotides. Hence these artificial cfDNA may not behave the same as real cfDNA. For example, artificial cfDNA having 3'-phosphate groups and/or 5'-hydroxyl groups may have varying degrees of ligation efficiencies that are not representative of *in vivo* cfDNA.

In view of the foregoing, there is a need for cell-free nucleic acid standards such as cfDNA standards that better represent *in vivo* cfDNA. The present disclosure addresses these needs, and provides further advantages as well.

In an aspect, a method for estimating abundance of a target nucleic acid present in a cell-free nucleic acid (CFNA) sample, defined in the appended claims, comprising the target nucleic acid and non-target nucleic acids comprises (a) quantifying copy number of the target nucleic acid in the CFNA sample to obtain an observed abundance of the target nucleic acid; (b) generating a calibration scheme by correlating an observed abundance of a reference nucleic acid present in a CFNA standard to an expected abundance of the reference nucleic acid present in the CFNA standard, which CFNA standard comprises a plurality of genomic polynucleotides, individual members of the plurality having a 5' terminal end and a 3' terminal end, wherein: (i) at least a subset of the plurality of genomic polynucleotides of the CFNA standard have a length ranging from about 100-300 bases; and (ii) a majority of the genomic polynucleotides of the CFNA standard have a phosphate group at a 5' terminal end and a hydroxyl group at a 3' terminal end, which majority of genomic polynucleotides are ligatable without generating a phosphate group at a 5' terminal end and/or generating a hydroxyl group at a 3' terminal end; and (c) estimating abundance of the target nucleic acid in the CFNA sample by adjusting the observed abundance of the target nucleic acid using the calibration scheme. In some embodiments, less than 50% of individual genomic polynucleotides of the CFNA standard have identical sequences. In some embodiments, the CFNA standard comprises at least one subset of genomic polynucleotides having identical members. In some embodiments, the subset represents less than 50%, 45%, 40%, 35%, 30%, 25%, 20%, 15%, 10%, or 5% of the CFNA standard. In some embodiments, at least 30% of the genomic polynucleotides have a length ranging from about 100-300 bases. In some embodiments, at least 50% of the genomic polynucleotides comprise a phosphate group at the 5' terminal end and a hydroxyl group at the 3' end. In some embodiments, a phosphate group at a 5' terminal end and a hydroxyl group at a 3' terminal end of a genomic polynucleotide are not generated by a polynucleotide kinase. In some embodiments, the genomic polynucleotides of the CFNA standard are ligatable with an efficiency of at least about 50%. In some embodiments, genomic polynucleotides of the CFNA standard are ligatable with an efficiency of at least about 50% as ascertained by a quantitative polymerase chain reaction (PCR) assay. In some embodiments, the genomic polynucleotides of the CFNA standard are ligatable in the absence of a phosphate donor.

In some embodiments, the expected abundance of the reference nucleic acid present in CFNA standard is less than 20%. In some embodiments, the reference nucleic acid comprises a mutant allele. In some embodiments, the mutant allele is present in the CFNA standard at an allelic frequency of less than 50%.

In some embodiments, generating the calibration scheme further comprises correlating an observed abundance of the reference nucleic acid present in an additional CFNA standard to an expected abundance of the reference nucleic acid present in the additional CFNA standard. In some embodiments, the expected abundance of the reference nucleic acid present in the additional CFNA standard is different from the expected abundance of the reference nucleic acid present in the CFNA standard.

In some embodiments, generating the calibration scheme further comprises correlating an observed abundance of at least one additional reference nucleic acid present in the CFNA standard to an expected abundance of the at least one additional reference nucleic acid present in the CFNA standard. In some embodiments, the expected abundance of the at least one additional reference nucleic acid present in the CFNA standard is less than 20%. In some embodiments, the at least one additional reference nucleic acid comprises an additional mutant allele. In some embodiments, the additional mutant allele is present in the CFNA standard at an allelic frequency of less than 50%.

In some embodiments, the CFNA standard comprises single-stranded genomic polynucleotides. In some embodiments, the CFNA standard comprises double-stranded genomic polynucleotides. In some embodiments, the double-stranded genomic polynucleotides are denatured to form single-stranded genomic polynucleotides prior to generating the calibration scheme.

In some embodiments, the calibration scheme comprises a calibration algorithm, which calibration algorithm adjusts for deviation of an observed abundance of the reference nucleic acid from an expected abundance of the reference nucleic acid for a plurality of expected abundance levels of the reference nucleic acid. In some embodiments, the calibration scheme comprises a line of best fit.

In some embodiments, the observed abundance of the target nucleic acid is determined by an amplification reaction. In some embodiments, the amplification reaction comprises digital polymerase chain reaction (dPCR). In some embodiments, the amplification reaction comprises droplet digital polymerase chain reaction (ddPCR). In some embodiments, the amplification reaction comprises quantitative polymerase chain reaction (qPCR). In some embodiments, the amplification reaction is performed with amplification primers specific to the target nucleic acid.

In some embodiments, the observed abundance of the target nucleic acid present in the CFNA sample is determined by (a) sequencing a plurality of amplification products to generate a plurality of sequence reads, wherein the plurality of amplification products are generated by amplifying the target nucleic acid and non-target nucleic acids of the CFNA sample; and (b) analyzing the sequence reads to calculate the observed abundance of the target nucleic acid.

In some embodiments, the target nucleic acid and the non-target nucleic acids are circularized to produce a plurality of circularized target nucleic acids and a plurality of circularized non-target nucleic acids prior to amplification. In some embodiments, circularizing is effected by subjecting the target nucleic acid and the non-target nucleic acids present in the CFNA sample to a ligation reaction. In some embodiments, the target nucleic acid and the non-target nucleic acids are circularized in the absence of a phosphate donor. In some embodiments, the target nucleic acid and the non-target nucleic acids are circularized with an efficiency of at least 50%. In some embodiments, the method further comprises degrading uncircularized target nucleic acid and uncircularized non-target nucleic acids prior to amplification.

In some embodiments, amplifying comprises rolling circle amplification. In some embodiments, amplifying comprises extension of random primers. In some embodiments, amplifying comprises extension of one or more primers specific to a target sequence. In some embodiments, the primers comprise a tag sequence, a sequencing primer binding sequence, or both.

In some embodiments, the target nucleic acid and the non-target nucleic acids are joined to adaptor polynucleotides to produce a plurality of adaptor-tagged target nucleic acids and a plurality of adaptor-tagged non-target nucleic acids prior to amplification. In some embodiments, joining to adaptor polynucleotides is effected by subjecting the target nucleic acid and the non-target nucleic acids to a ligation reaction. In some embodiments, the target nucleic acid and the non-target nucleic acids are ligated to adaptor polynucleotides in the absence of a phosphate donor. In some embodiments, the target nucleic acid and the non-target nucleic acids are ligated to adaptor polynucleotides with an efficiency of at least 50%. In some embodiments, the target nucleic acid and the non-target nucleic acids are ligated to adaptor polynucleotides after A-tailing with an efficiency of at least 50%.

In some embodiments, an adaptor polynucleotide comprises a tag sequence, a sequencing primer binding sequence, or both. In some embodiments, amplifying comprises extension of one or more primers specific to a primer binding sequence of the adaptor polynucleotide.

In some embodiments, the target nucleic acid comprises a nucleotide sequence that is at least 90% identical to a nucleotide sequence of the reference nucleic acid. In some embodiments, the estimated abundance of the target nucleic acid is a concentration. In some embodiments, the target nucleic acid comprises a mutant allele. In some embodiments, the estimated abundance of the target nucleic acid is an allelic frequency.

In an aspect, a method, which is not claimed, for assessing a detection limit of a cell-free nucleic acid (CFNA) assay comprises (a) performing the CFNA assay with a plurality of CFNA standards to obtain for each standard an observed abundance of a reference polynucleotide present in each standard, said plurality of CFNA standards covering a given range of expected abundances of the reference polynucleotide, wherein each standard of the plurality has an expected abundance of the reference polynucleotide that is different from that of other standards of the plurality, and wherein each standard comprises a plurality of genomic polynucleotides, individual members of the plurality having a 5' terminal end and a 3' terminal end, wherein: (i) for an individual standard of the plurality, at least a subset of the plurality of genomic polynucleotides have a length ranging from about 100-300 bases; and (ii) for an individual standard of the plurality, a majority of the genomic polynucleotides have a phosphate group at a 5' terminal end and a hydroxyl group at a 3' terminal end, which majority of genomic polynucleotides are ligatable without generating a phosphate group at a 5' terminal end and/or generating a hydroxyl group at a 3' terminal end; and (b) identifying an expected abundance at which a corresponding observed abundance of the reference polynucleotide is statistically indistinguishable from a background measurement, thereby calling the expected abundance as the detection limit of the CFNA assay. In some embodiments, the given range of expected abundances of the reference polynucleotide is from about 0.001% to 20%. In some embodiments, the reference polynucleotide comprises a mutant allele. In some embodiments, the expected abundance is an allelic frequency. In some embodiments, the range of allelic frequencies is from about 0.001% to 50%.

In some embodiments, less than 50% of individual genomic polynucleotides of a given standard CFNA standard of the plurality have identical sequences. In some embodiments, at least one of the plurality of CFNA standards comprises at least one subset of genomic polynucleotides having identical members. In some embodiments, the subset represents less than 50%, 45%, 40%, 35%, 30%, 25%, 20%, 15%, 10%, or 5% of the CFNA standard. In some embodiments, at least 30% of the genomic polynucleotides of a given CFNA standard of the plurality have a length ranging from about 100-300 bases. In some embodiments, at least 50% of the genomic polynucleotides of a given CFNA standard of the plurality comprise a phosphate group at the 5' terminal end and a hydroxyl group at the 3' end. In some embodiments, a phosphate group at a 5' terminal end and a hydroxyl group at a 3' terminal end of a genomic polynucleotide are not generated by a polynucleotide kinase. In some embodiments, the genomic polynucleotides of a given CFNA standard of the plurality are ligatable with an efficiency of at least 50%. In some embodiments, the genomic polynucleotides of a given CFNA standard of the plurality are ligatable in the absence of a phosphate donor.

In some embodiments, the CFNA assay comprises circularizing the genomic polynucleotides of the CFNA standards to produce a plurality of circularized genomic polynucleotides. In some embodiments, circularizing is effected by subjecting the genomic polynucleotides to a ligation reaction. In some embodiments, the genomic polynucleotides are circularized with an efficiency of at least 50%.

In some embodiments, the CFNA assay comprises joining genomic polynucleotides to adaptor polynucleotides to produce a plurality of adaptor tagged genomic polynucleotides. In some embodiments, joining genomic polynucleotides to adaptor polynucleotides is effected by subjecting the genomic polynucleotides to a ligation reaction. In some embodiments, the genomic polynucleotides are ligated to adaptor polynucleotides with an efficiency of at least 50%. In some embodiments, the genomic polynucleotides are ligated to adaptor polynucleotides after A-tailing with a ligation efficiency of at least 50%.

In some embodiments, a given CFNA standard of the plurality comprises single-stranded genomic polynucleotides. In some embodiments, a given CFNA standard of the plurality comprises double-stranded genomic polynucleotides. In some embodiments, the CFNA assay comprises next generation sequencing (NGS), digital polymerase chain reaction (dPCR), droplet digital polymerase chain reaction (ddPCR), and/or quantitative polymerase chain reaction (qPCR).

In an aspect, a method, which is not claimed, for assessing sensitivity of a cell-free nucleic acid (CFNA) assay for detecting reference polynucleotides present in a CFNA standard at a given expected abundance comprises (a) performing the CFNA assay with a CFNA standard comprising a plurality of reference polynucleotides to obtain a positive or negative detection call for each reference polynucleotide of the plurality, wherein each reference polynucleotide is present in the CFNA standard at the given expected abundance, wherein the CFNA standard comprises a plurality of genomic polynucleotides, individual members of the plurality having a 5' terminal end and a 3' terminal end, and wherein: (i) at least a subset of the plurality of genomic polynucleotides of the CFNA standard have a length ranging from about 100-300 bases; and (ii) a majority of the genomic polynucleotides of the CFNA standard have a phosphate group at a 5' terminal end and a hydroxyl group at a 3' terminal end, which majority of genomic polynucleotides are ligatable without generating a phosphate group at a 5' terminal end and/or generating a hydroxyl group at a 3' terminal end; and (b) determining the fraction of reference polynucleotides yielding a positive detection call, thereby assessing the sensitivity of the CFNA assay for detecting reference polynucleotides present in the CFNA standard at the given expected abundance.

In an aspect, a method, which is not claimed, for assessing specificity of a cell-free nucleic acid (CFNA) assay comprises (a) performing the CFNA assay with a CFNA standard to obtain a positive or negative detection call for each of a plurality of reference polynucleotides, wherein each reference polynucleotide is absent in the CFNA standard, and wherein: (i) at least a subset of the plurality of genomic polynucleotides of the CFNA standard have a length ranging from about 100-300 bases; and (ii) a majority of the genomic polynucleotides of the CFNA standard have a phosphate group at a 5' terminal end and a hydroxyl group at a 3' terminal end, which majority of genomic polynucleotides are ligatable without generating a phosphate group at a 5' terminal end and/or generating a hydroxyl group at a 3' terminal end; and (b) determining the fraction of reference polynucleotides yielding a negative detection call, thereby assessing the specificity of the CFNA assay.

In some embodiments, less than 50% of individual genomic polynucleotides of the CFNA standard have identical sequences. In some embodiments, the CFNA standard comprises at least one subset of genomic polynucleotides having identical members. In some embodiments, the subset represents less than 50%, 45%, 40%, 35%, 30%, 25%, 20%, 15%, 10%, or 5% of the CFNA standard. In some embodiments, at least 30% of the genomic polynucleotides have a length ranging from about 100-300 bases. In some embodiments, at least 50% of the genomic polynucleotides comprise a phosphate group at the 5' terminal end and a hydroxyl group at the 3' end. In some embodiments, a phosphate group at a 5' terminal end and a hydroxyl group at a 3' terminal end of a genomic polynucleotide are not generated by a polynucleotide kinase. In some embodiments, the genomic polynucleotides of the CFNA standard are ligatable with an efficiency of at least 50%. In some embodiments, the genomic polynucleotides of the CFNA standard are ligatable in the absence of a phosphate donor.

In some embodiments, the given expected abundance is less than 20%. In some embodiments, each reference polynucleotide of the CFNA standard comprises a mutant allele. In some embodiments, each mutant allele is present in the CFNA standard at an allelic frequency of less than 50%. In some embodiments, the plurality of reference polynucleotides comprises at least two reference polynucleotides.

In some embodiments, the CFNA assay comprises circularizing the genomic polynucleotides of the CFNA standard to produce a plurality of circularized genomic polynucleotides. In some embodiments, circularizing is effected by subjecting the genomic polynucleotides to a ligation reaction. In some embodiments, the genomic polynucleotides are circularized with an efficiency of at least 50%.

In some embodiments, the CFNA assay comprises joining genomic polynucleotides to adaptor polynucleotides to produce a plurality of adaptor tagged genomic polynucleotides. In some embodiments, joining genomic polynucleotides to adaptor polynucleotides is effected by subjecting the genomic polynucleotides to a ligation reaction. In some embodiments, the genomic polynucleotides are ligated to adaptor polynucleotides with an efficiency of at least 50%. In some embodiments, the genomic polynucleotides are ligated to adaptor polynucleotides after A-tailing with a ligation efficiency of at least 50%.

In some embodiments, the CFNA standard comprises single-stranded genomic polynucleotides. In some embodiments, the CFNA standard comprises double-stranded genomic polynucleotides. In some embodiments, the CFNA assay comprises next generation sequencing (NGS), digital polymerase chain reaction (dPCR), droplet digital polymerase chain reaction (ddPCR), and/or quantitative polymerase chain reaction (qPCR).

In an aspect, a method, which is not claimed, of developing a cell-free nucleic acid (CFNA) assay comprises (a) performing the CFNA assay with a CFNA standard under a plurality of assay conditions to yield a set of performance metrics, wherein the CFNA standard is associated with a set of reference performance metrics when utilized in a reference CFNA assay, wherein the CFNA standard comprises a plurality of genomic polynucleotides, individual members of the plurality having a 5' terminal end and a 3' terminal end, wherein: (i) at least a subset of the plurality of genomic polynucleotides of the CFNA standard have a length ranging from about 100-300 bases; and (ii) a majority of the genomic polynucleotides of the CFNA standard have a phosphate group at a 5' terminal end and a hydroxyl group at a 3' terminal end, which majority of the genomic polynucleotides are ligatable without generating a phosphate group at a 5' terminal end and/or generating a hydroxyl group at a 3' terminal end; and (b) adjusting one or more assay conditions to improve at least one performance metric relative to at least one reference performance metric, thereby developing the CFNA assay.

In some embodiments, at least one performance metric is a detection rate (observed abundance/expected abundance) or a limit of detection. In some embodiments, the assay comprises a ligation reaction. In some embodiments, an assay condition is ligation time or ligation temperature. In some embodiments, the assay comprises an amplification reaction. In some embodiments, an assay condition is an amplification temperature, length of an amplification step, or number of amplification cycles.

In some embodiments, the standard comprises at least one reference nucleic acid. In some embodiments, the at least one reference nucleic acid is present in the CFNA standard at an expected abundance of less than 20%. In some embodiments, the at least one reference nucleic acid comprises a mutant allele. In some embodiments, the mutant allele is present in the CFNA standard at an allelic frequency of less than 50%.

In an aspect, a system for estimating abundance of a target nucleic acid in a cell-free nucleic acid (CFNA) sample comprising the target nucleic acid and non-target nucleic acids comprises a quantification system configured to determine copy number of the target nucleic acid in the CFNA sample to yield an observed abundance of the target nucleic acid; a computer configured to (a) generate a calibration scheme by correlating an observed abundance of a reference nucleic acid present in a CFNA standard to an expected abundance of the reference nucleic acid present in the CFNA standard, which CFNA standard comprises a plurality of genomic polynucleotides, individual members of the plurality having a 5' terminal end and a 3' terminal end, wherein: (i) at least a subset of the plurality of genomic polynucleotides of the CFNA standard have a length ranging from about 100-300 bases; and (ii) a majority of the genomic polynucleotides of the CFNA standard have a phosphate group at a 5' terminal end and a hydroxyl group at a 3' terminal end, which majority of the genomic polynucleotides are ligatable without generating a phosphate group at a 5' terminal end and/or generating a hydroxyl group at a 3' terminal end; and (b) estimate abundance of the target nucleic acid in the CFNA sample by adjusting the observed abundance of the target nucleic acid using the calibration scheme. In some embodiments, the system further comprises a report generator that sends a report to a recipient, wherein the report contains at least one of the following: observed abundance of the target nucleic acid, estimated abundance of the target nucleic acid, observed abundance of the reference nucleic acid, expected abundance of the reference nucleic acid, and calibration scheme.

In some embodiments, a computer-readable medium, which is not claimed, comprising code that, upon execution by one or more processors, implements a method for estimating abundance of a target nucleic acid in a cell-free nucleic acid (CFNA) sample comprising the target nucleic acid and non-target nucleic acids, the method comprises (a) in response to a user request, performing a quantification reaction to determine copy number of the target nucleic acid in the CFNA sample and yield an observed abundance of the target nucleic acid; (b) generating a calibration scheme by correlating an observed abundance of a reference nucleic acid present in a CFNA standard to an expected abundance of the reference nucleic acid present in the CFNA standard, which CFNA standard comprises a plurality of genomic polynucleotides, individual members of the plurality having a 5' terminal end and a 3' terminal end, wherein: (i) at least a subset of the plurality of genomic polynucleotides of the CFNA standard have a length ranging from about 100-300 bases; and (ii) a majority of the genomic polynucleotides of the CFNA standard have a phosphate group at a 5' terminal end and a hydroxyl group at a 3' terminal end, which majority of the genomic polynucleotides are ligatable without generating a phosphate group at a 5' terminal end and/or generating a hydroxyl group at a 3' terminal end; and (c) estimating abundance of the target nucleic acid in the CFNA sample by adjusting the observed abundance of the target nucleic acid using the calibration scheme. In some embodiments, the method further comprises (d) generating a report that contains at least one of the following: observed abundance of the target nucleic acid, estimated abundance of the target nucleic acid, observed abundance of the reference nucleic acid, expected abundance of the reference nucleic acid, and calibration scheme.

In an aspect, a kit, which is not claimed, comprises (a) a cell-free nucleic acid (CFNA) standard comprising a plurality of genomic polynucleotides, individual members of the plurality having a 5' terminal end and a 3' terminal end, wherein: (i) at least a subset of the plurality of genomic polynucleotides of the CFNA standard have a length ranging from about 100-300 bases; and (ii) a majority of the genomic polynucleotides of the CFNA standard have a phosphate group at a 5' terminal end and a hydroxyl group at a 3' terminal end, which majority of the genomic polynucleotides are ligatable without generating a phosphate group at a 5' terminal end and/or generating a hydroxyl group at a 3' terminal end; and (b) user instructions for using the CFNA standard in a CFNA analysis.

In some embodiments, less than 50% of individual genomic polynucleotides of the CFNA standard have identical sequences. In some embodiments, the CFNA standard of the kit comprises at least one subset of genomic polynucleotides having identical members. In some embodiments, the subset represents less than 50%, 45%, 40%, 35%, 30%, 25%, 20%, 15%, 10%, or 5% of the CFNA standard. In some embodiments, at least 30% of the genomic polynucleotides have a length ranging from about 100-300 bases. In some embodiments, at least 50% of the genomic polynucleotides comprise a phosphate group at the 5' terminal end and a hydroxyl group at the 3' end. In some embodiments, a phosphate group at a 5' terminal end and a hydroxyl group at a 3' terminal end of a genomic polynucleotide are not generated by a polynucleotide kinase. In some embodiments, the genomic polynucleotides of the CFNA standard are ligatable with an efficiency of at least 50%. In some embodiments, the genomic polynucleotides of the CFNA standard are ligatable in the absence of a phosphate donor.

In some embodiments, the CFNA standard comprises a reference nucleic acid. In some embodiments, the reference nucleic acid is present in the CFNA standard at an expected abundance of less than 20%. In some embodiments, the reference nucleic acid comprises a mutant allele. In some embodiments, the mutant allele is present in the CFNA standard at an allelic frequency of less than 50%. In some embodiments, the CFNA standard comprises a plurality of reference nucleic acids. In some embodiments, each reference nucleic acid of the plurality comprises a mutant allele. In some embodiments, each mutant allele is present at an allelic frequency of less than 50%.

In some embodiments, the CFNA standard comprises single-stranded genomic polynucleotides. In some embodiments, the CFNA standard comprises double-stranded genomic polynucleotides.

In some embodiments, the kit further comprises a ligase. In some embodiments, the kit further comprises a ligation reaction buffer.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
FIG. 1A shows Bioanalyzer results of a control cfDNA sample.
FIG. 1B shows Bioanalyzer results of a cell-free nucleic acid (CFNA) standard.
FIG. 2 shows the results from electrophoresis of (i) untreated, (ii) ligase treated, and (iii) ligase + exonuclease treated control cfDNA, CFNA standard, and sonicated genomic DNA samples.

### DETAILED DESCRIPTION OF THE INVENTION

The practice of some methods disclosed herein employ, unless otherwise indicated, conventional techniques of immunology, biochemistry, chemistry, molecular biology, microbiology, cell biology, genomics and recombinant DNA, which are within the skill of the art. See for example Sambrook and Green, Molecular Cloning: A Laboratory Manual, 4th Edition (2012); the series Current Protocols in Molecular Biology (F. M. Ausubel, et al. eds.); the series Methods In Enzymology (Academic Press, Inc.), PCR 2: A Practical Approach (M.J. MacPherson, B.D. Hames and G.R. Taylor eds. (1995)), Harlow and Lane, eds. (1988) Antibodies, A Laboratory Manual, and Culture of Animal Cells: A Manual of Basic Technique and Specialized Applications, 6th Edition (R.I. Freshney, ed. (2010)).

As used in the specification and claims, the singular forms "a", "an" and "the" include plural references unless the context clearly dictates otherwise. For example, the term "a target nucleic acid" includes a plurality of target nucleic acids, including mixtures thereof.

The term "about" or "approximately" means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated, the term "about" meaning within an acceptable error range for the particular value should be assumed.

The terms "polynucleotide", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA (tRNA), ribosomal RNA (rRNA), short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, cell-free polynucleotides including cell-free DNA (cfDNA) and cell-free RNA (cfRNA), nucleic acid probes, and primers. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

A polynucleotide may have a 5' end and 3' end, referring to the end-to-end chemical orientation of a single strand of polynucleotide or nucleic acid. In a single strand of linear DNA or RNA, the chemical convention of naming carbon atoms in the nucleotide sugar-ring means that there generally exists a 5' end which frequently contains a phosphate group attached to the 5' carbon and a 3' end which typically is unmodified from the ribose -OH substituent (hydroxyl group). In some cases, a polynucleotide may have a -OH substituent or a hydroxyl group at a 5' end and -P group or phosphate group at a 3' end. A phosphate group attached to the 5'-end permits ligation of two nucleotides, e.g., the covalent binding of a 5'-phosphate to the 3'-hydroxyl group of another nucleotide, to form a phosphodiester bond. Removal of the 5'-phosphate may inhibit or prevent ligation. The 3'-hydroxyl group is also important as it is joined to the 5'-phosphate in ligation.

The term "cell-free nucleic acid" or "CFNA" refers to extracellular nucleic acids. Extracellular nucleic acids can be found in biological sources such as blood, urine, and stool. CFNA may refer to cell-free DNA (cfDNA), cell-free RNA (cfRNA), or both. CFNA may result from the shedding of nucleic acids from cells undergoing apoptosis or necrosis. Previous studies have demonstrated that CFNA, for example cfDNA, exists at steady-state levels and can increase with cellular injury or necrosis. In some cases, CFNA is shed from abnormal cells or unhealthy cells, such as tumor cells. cfDNA shed from tumor cells, in some cases, can be distinguished from cfDNA shed from normal or healthy cells using genomic information, such as by identifying genetic variations including mutations and/or gene fusions distinguishing between normal and abnormal cells. In some cases, CFNA is shed from cells associated with a fetus into maternal circulation. In some cases, CFNA may originate from a pathogen that has infected a host, such as a subject (e.g., patient).

The term "genomic polynucleotide," as used herein, refers to a polynucleotide derived or isolated from a chromosome. A genomic polynucleotide may refer to a contiguous portion of a chromosome of any length.

The terms "target polynucleotide" and "target nucleic acid," as used herein, refer to a nucleic acid molecule or polynucleotide in a starting population of nucleic acid molecules having a target sequence whose presence, amount, and/or nucleotide sequence, or changes in one or more of these, are desired to be determined. The target polynucleotide may be a portion of a larger polynucleotide (e.g. a portion to be amplified, sequenced, or otherwise analyzed), or may be used to refer to the larger polynucleotide comprising a target sequence. In general, the term "target sequence" refers to a nucleic acid sequence on a single strand of nucleic acid. The target sequence may be a portion of a gene, a regulatory sequence, genomic DNA, CFNA including cfDNA and/or cfRNA, cDNA, fusion gene, RNA including mRNA, miRNA, rRNA, or others. The target sequence may be a target sequence from a sample or a secondary target such as a product of an amplification reaction. The target sequence may comprise an allele (e.g., wild-type or variant allele).

The term "allele," as used herein, refers to any of one or more alternative forms of a gene at a particular locus, all of which may relate to one trait or characteristic at the specific locus. In a diploid cell of an organism, alleles of a given gene can be located at a specific location, or locus (loci plural) on a chromosome. The sequences at these variant sites that differ between different alleles are termed "variants", "polymorphisms", or "mutations".

The term "wild-type" when made in reference to an allele or sequence, generally refers to the allele or sequence that encodes the phenotype most common in a particular natural population. In some cases, a wild-type allele can refer to an allele present at highest frequency in the population. In some cases, a wild-type allele or sequence refers to an allele or sequence associated with a normal state relative to an abnormal state, for example a disease state.

The term "mutant" or "variant," when made in reference to an allele or sequence, generally refers to an allele or sequence that does not encode the phenotype most common in a particular natural population. In some cases, a mutant allele can refer to an allele present at a lower frequency in a population relative to the wild-type allele. In some cases, a mutant allele or sequence can refer to an allele or sequence mutated from a wild-type sequence to a mutated sequence that presents a phenotype associated with a disease state. Mutant alleles and sequences may be different from wild-type alleles and sequences by only one base, but can be different up to several bases. The term mutant when made in reference to a gene generally refers to one or more sequence mutations in a gene, including a point mutation, a single nucleotide polymorphism (SNP), an insertion, a deletion, a substitution, a transposition, a translocation, a copy number variation, or another genetic mutation, alteration or sequence variation.

The terms "allele frequency" or "allelic frequency," as used herein, generally refer to the relative frequency of an allele (e.g., variant of a gene) in a sample, e.g., expressed as a fraction or percentage. In some cases, allelic frequency may refer to the relative frequency of an allele (e.g., variant of a gene) in a sample, such as a cell-free nucleic acid sample. In some cases, allelic frequency may refer to the relative frequency of an allele (e.g., variant of a gene) in a sample, such as a cell-free nucleic acid standard. The allelic frequency of a mutant allele may refer to the frequency of the mutant allele relative to the wild-type allele in a sample, e.g., a cell-free nucleic acid sample. For example, if a sample comprises 100 copies of a gene, five of which are a mutant allele and 95 of which are the wild-type allele, an allelic frequency of the mutant allele is about 5/100 or about 5%. A sample having no copies of a mutant allele (e.g., about 0% allelic frequency) may be used, for example, as a negative control. A negative control may be a sample in which no mutant allele is expected to be detected. A sample comprising a mutant allele at about 50% allelic frequency may, for example, be representative of a germline heterozygous mutation.

In general, the term "sequence variant" refers to any variation in sequence relative to one or more reference sequences. Typically, the sequence variant occurs with a lower frequency than the reference sequence for a given population of individuals for whom the reference sequence is known. In some cases, the reference sequence is a single known reference sequence, such as the genomic sequence of a single individual. In some cases, the reference sequence is a consensus sequence formed by aligning multiple known sequences, such as the genomic sequence of multiple individuals serving as a reference population, or multiple sequencing reads of polynucleotides from the same individual. In some cases, the sequence variant occurs with a low frequency in the population (also referred to as a "rare" sequence variant). For example, the sequence variant may occur with a frequency of about or less than about 5%, 4%, 3%, 2%, 1.5%, 1%, 0.75%, 0.5%, 0.25%, 0.1%, 0.075%, 0.05%, 0.04%, 0.03%, 0.02%, 0.01%, 0.005%, 0.001%, or lower. In some cases, the sequence variant occurs with a frequency of about or less than about 0.1%. A sequence variant can be any variation with respect to a reference sequence. A sequence variation may consist of a change in, insertion of, or deletion of a single nucleotide, or of a plurality of nucleotides (e.g. 2, 3, 4, 5, 6, 7, 8, 9, 10, or more nucleotides). Where a sequence variant comprises two or more nucleotide differences, the nucleotides that are different may be contiguous with one another, or discontinuous. Non-limiting examples of types of sequence variants include single nucleotide polymorphisms (SNP), deletion/insertion polymorphisms (DIP), copy number variants (CNV), short tandem repeats (STR), simple sequence repeats (SSR), variable number of tandem repeats (VNTR), amplified fragment length polymorphisms (AFLP), retrotransposon-based insertion polymorphisms, sequence specific amplified polymorphism, and differences in epigenetic marks that can be detected as sequence variants (e.g. methylation differences). In some embodiments, a sequence variant can refer to a chromosome rearrangement, including but not limited to a translocation or fusion gene.

The term "observed abundance," as used herein, generally refers to the relative representation of, for example, a particular species (e.g., target nucleic acid) in a sample (e.g., nucleic acid sample) that is observed, detected, or measured. For example, an observed abundance may refer to the relative representation of a target polynucleotide in a polynucleotide sample that is observed or detected, for example by an assay such as a cell-free nucleic acid assay (CFNA assay). This may be, for example, the number of target polynucleotide molecules relative to the total number of polynucleotides of the polynucleotide sample. If the target polynucleotide comprises a mutant allele or variant allele, an observed abundance may refer to the observed, detected or measured allelic frequency of the mutant in a sample. Abundance may be described as a fraction or percentage, for example of the total or a subset of the total (e.g., mutant allele relative to all alleles, including wild-type and other mutants, present in a sample). An abundance may, in some cases, be described as a concentration including, but not limited to, mass concentration, molar concentration, number concentration, and volume concentration, or other acceptable unit of measure.

The term "expected abundance," as used herein, generally refers to the relative representation of, for example, a particular species (e.g., target nucleic acid) in a sample (e.g., nucleic acid sample) that is expected to be characteristic of the sample. For example, an expected abundance may refer to the relative representation of a target polynucleotide in a polynucleotide sample that is expected to be characteristic the sample (e.g., a reference sample or standard sample). The relative representation of a target polynucleotide in a polynucleotide sample may be expected, for example, if the sample was artificially generated by spiking in or adding known amounts of a target polynucleotide to a polynucleotide sample (the amounts are also known). Abundance may be described as a fraction or percentage, for example of the total (e.g., number of target polynucleotide molecules relative to total number of polynucleotide molecules in the sample) or a subset of the total (e.g., number of molecules of a mutant allele relative to the total number of molecules of the gene, including mutant and wild-type alleles). Abundance may, in some cases, be described as a concentration including, but not limited to, mass concentration, molar concentration, number concentration, and volume concentration, or other acceptable unit of measure.

The term "estimated abundance," as used herein, generally refers to an estimate of the relative representation of, for example, a particular species (e.g., target nucleic acid) in a sample (e.g., cell-free nucleic acid sample). An estimated abundance may be a value obtained from adjusting an observed abundance by a calibration or correction scheme which accounts for variability or error in a measurement method and/or system. Where a measurement method or system has little variability and/or error and is highly sensitive, specific, and/or accurate, an estimated abundance and an observed abundance may deviate insignificantly. Where a measurement method or system has high variability and/or low sensitivity, specificity, and/or accuracy, an estimated abundance and an observed abundance may differ significantly. Abundance may be described as a fraction or percentage, for example of the total (e.g., number of target polynucleotide molecules relative to total number of polynucleotide molecules) or a subset of the total (e.g., number of molecules of a mutant allele relative to the total number of molecules of the gene, including mutant and wild-type alleles). Abundance may, in some cases, be described as a concentration including, but not limited to, mass concentration, molar concentration, number concentration, and volume concentration, or other acceptable unit of measure.

The terms "standard" or "reference," as used herein, generally refers to a substance which is prepared to certain pre-defined criteria and can be used to assess certain aspects of, for example, an assay. Standards or references preferably yield reproducible, consistent and reliable results. These aspects may include performance metrics, examples of which include, but are not limited to, accuracy, specificity, sensitivity, linearity, reproducibility, and limit of detection or limit of quantitation. Standards or references may be used for assay development, assay validation, and/or assay optimization. Standards may be used to evaluate quantitative and qualitative aspects of an assay.

The term "limit of detection," as used herein, generally refers to the lowest quantity or amount of a substance that can be detected with reasonable certainty for a given procedure (e.g., assay or analysis). A limit of detection may refer to an instrument detection limit and/or a method detection limit, wherein an instrument detection limit is associated with the inherent limitations in the operation of an instrument or device and a method detection limit is associated with performing the steps of a particular method.

The terms "ligate" and "ligation," as used herein, refer to any enzymatic or non-enzymatic process by which an inter-nucleotide linkage is formed between two polynucleotide ends, which ends optionally are adjacently hybridized to a template. For example, the ends of DNA fragments can be ligated by forming a phosphodiester bond between the 3'-hydroxyl of one DNA terminus with the 5'-phosphoryl of another. In some cases, the inter-nucleotide linkage is formed between two polynucleotide fragments (intermolecular). In some cases, the inter-nucleotide linkage is formed between two terminal ends (5' end and 3' end) of a single fragment (intramolecular). Terminal ends of RNA fragments can similarly be joined by the formation of a phosphodiester bond. Polynucleotides that can be ligated may either be single-stranded or double-stranded. Double-stranded nucleic acids can comprise staggered ends, overhangs, or sticky ends where there are unpaired nucleotides at the 3' or 5' end of the DNA or RNA molecule. Double-stranded nucleic acids can comprise blunt ends, where the end nucleotides are paired at the 3' or 5' end of the DNA or RNA molecule. Ligation can comprise use of an enzyme, such as a ligase enzyme.

The terms "hybridize," "hybridization," "hybridizing," "anneal," and "annealing," as used herein, generally refer to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self-hybridizing strand, or any combination of these. A hybridization reaction may constitute a step in a more extensive process, such as the initiation of a PCR, or the enzymatic cleavage of a polynucleotide by a ribozyme. A first sequence that can be stabilized via hydrogen bonding with the bases of the nucleotide residues of a second sequence is said to be "hybridizable" to the second sequence. In such a case, the second sequence can also be said to be hybridizable to the first sequence.

The terms "amplify," "amplifies," "amplified," "amplification," as used herein, generally refer to any process by which one or more copies are made of a target polynucleotide or a portion thereof. A variety of methods of amplifying polynucleotides (e.g. DNA and/or RNA) are available, some examples of which are described herein. Amplification may be linear, exponential, or involve both linear and exponential phases in a multi-phase amplification process. Amplification methods may involve changes in temperature, such as a heat denaturation step, or may be isothermal processes that do not require heat denaturation.

The term "primer," as used herein, refers to a single-stranded oligonucleotide capable of acting as a point of initiation for template-directed DNA synthesis under suitable conditions for example, buffer and temperature, in the presence of at least one nucleoside triphosphate and an agent for polymerization, such as, for example, DNA or RNA polymerase or reverse transcriptase. The length of the primer, in any given case, depends on, for example, the intended use of the primer, and typically ranges from about 10 to 30 nucleotides. Short primer molecules generally require cooler temperatures to form sufficiently stable hybridized complexes with the template. A primer need not reflect the exact sequence of the template but must be sufficiently complementary to hybridize with such template. The primer site is the area of the template to which a primer hybridizes. A primer pair is a set of primers including a 5' upstream primer that hybridizes with the 5' end of the sequence to be amplified and a 3' downstream primer that hybridizes with the complement of the 3' end of the sequence to be amplified.

The terms "complement," "complements," "complementary," and "complementarity," as used herein, generally refer to a sequence that is fully complementary to and hybridizable to the given sequence. In some cases, a sequence hybridized with a given nucleic acid is referred to as the "complement" or "reverse-complement" of the given molecule if its sequence of bases over a given region is capable of complementarily binding those of its binding partner, such that, for example, A-T, A-U, G-C, and G-U base pairs are formed. In general, a first sequence that is hybridizable to a second sequence is specifically or selectively hybridizable to the second sequence, such that hybridization to the second sequence or set of second sequences is preferred (e.g. thermodynamically more stable under a given set of conditions, such as stringent conditions commonly used in the art) to hybridization with non-target sequences during a hybridization reaction. Typically, hybridizable sequences share a degree of sequence complementarity over all or a portion of their respective lengths, such as between 25%-100% complementarity, including at least 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, and 100% sequence complementarity. Sequence identity, such as for the purpose of assessing percent complementarity, may be measured by any suitable alignment algorithm, including but not limited to the Needleman-Wunsch algorithm (see e.g. the EMBOSS Needle aligner available at www.ebi.ac.uk/Tools/psa/emboss_needle/nucleotide.html, optionally with default settings), the BLAST algorithm (see e.g. the BLAST alignment tool available at blast.ncbi.nlm.nih.gov/Blast.cgi, optionally with default settings), or the Smith-Waterman algorithm (see e.g. the EMBOSS Water aligner available at www.ebi.ac.uk/Tools/psa/emboss_water/nucleotide.html, optionally with default settings). Optimal alignment may be assessed using any suitable parameters of a chosen algorithm, including default parameters. In some embodiments, a capture probe specifically hybridizes to a specified target sequence via complementarity between a pre-determined, non-random sequence of the capture probe and the target sequence.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained in vivo or cultured in vitro are also encompassed.

In various aspects, the disclosure provides compositions and kits comprising cell-free nucleic acid (CFNA) standards and methods of using a cell-free nucleic acid standard. CFNA generally refers to extracellular nucleic acids that can be obtained, for example, from serum, plasma, blood, perspiration, saliva, urine, stool, semen, mucosal excretions, spinal fluid, amniotic fluid, and lymph fluid (e.g., *in vivo* CFNA) of any subject, such as humans, non-human primates, rodents such as mice and rats, dogs, cats, pigs, sheep, rabbits and others. A cell-free nucleic acid present in a non-cellular source can result from cell death (e.g., apoptosis or necrosis) or cell shedding. Analyzing CFNA may be useful in characterizing the cell or population of cells from which the CFNA is derived, such as tumor cells (e.g. in cancer detection), fetal cells (e.g. in prenatal diagnostics), cells from transplanted tissue (e.g. in early detection of transplant failure), or a pathogen (e.g., bacteria or virus). In some subjects, *in vivo* CFNA comprises a mixture of nucleic acids from normal, healthy cells of a subject and nucleic acids derived from tumor cells (or other unhealthy tissue), nucleic acids derived from a fetus, nucleic acids derived from a non-autologous source (e.g., cell or tissue transplant), and/or a pathogen (e.g., bacteria or virus). CFNA derived from a tumor cell can, in some cases, be distinguished from CFNA derived from a non-tumor cell based on nucleotide sequence. For example, a tumor cell may possess a mutant allele at a particular gene whereas the corresponding gene of a normal cell possesses a wild-type allele. A CFNA having the sequence of the mutant allele may, for example, indicate that the particular nucleic acid is derived from a tumor cell while a CFNA having the sequence of the wild-type allele may, for example, indicate that that particular nucleic acid is derived from a normal cell. Similarly, fetal cells, non-autologous cells of a transplant, and pathogens may be distinguished from normal, healthy cells of a subject.

In some embodiments, a CFNA standard, which per se not claimed, disclosed herein is useful for developing a CFNA assay, validating a CFNA assay, optimizing a CFNA assay, and/or evaluating the performance of a CFNA assay, for example a CFNA assay to detect tumor nucleic acids, fetal nucleic acids, non-autologous nucleic acids of transplanted cells, and/or pathogenic nucleic acids. In some embodiments, a CFNA standard disclosed herein is useful to validate, verify, and/or normalize the results obtained from a CFNA assay, for example a CFNA assay to detect tumor nucleic acids, fetal nucleic acids, non-autologous nucleic acids of transplanted cells, and/or pathogenic nucleic acids.

A CFNA standard, which is per se not claimed, disclosed herein may possess properties or characteristics similar to and/or representative of that of naturally occurring cell-free nucleic acids, such as cell-free DNA isolated from a biological sample of a subject (e.g., *in vivo* CFNA). CFNA standards that possess properties or characteristics similar to and/or representative of that of *in vivo* CFNA, for example cell-free DNA (cfDNA), may be useful in CFNA assays. A CFNA standard disclosed herein may, in some cases, behave more similarly to *in vivo* CFNA in enzymatic reactions, including but not limited to intermolecular and/or intramolecular ligation, compared to a CFNA standard having nucleic acids having a physical and/or biochemical properties less similar to *in vivo* CFNA. For example, a CFNA standard further described herein may comprise nucleic acids having lengths reflective of the size distribution of CFNA obtained from a biological sample (e.g., *in vivo* CFNA). In addition to nucleic acid length, nucleic acids of a CFNA standard herein may mimic certain biological characteristics of *in vivo* CFNA. For example, a majority of the nucleic acids of a CFNA standard disclosed herein may comprise a phosphate group at a 5' terminal end and a hydroxyl group at a 3' terminal end.

In an aspect, the disclosure provides a cell-free nucleic acid (CFNA) standard, which is per se not claimed, comprising a plurality of genomic polynucleotides. Individual members of the plurality of genomic polynucleotides have a 5' terminal and a 3' terminal end. In some embodiments, at least a subset of the plurality of genomic polynucleotides have a length ranging from about 100-300 bases. In some embodiments, a majority of the genomic polynucleotides of the standard have a phosphate group at a 5' terminal end and hydroxyl group at a 3' terminal end and can be ligated, for example intramolecularly and/or intermolecularly, without the need for generating a phosphate group at a 5' terminal end and/or generating a hydroxyl group at a 3' terminal end.

Fragment sizes of *in vivo* cfDNA are generally centered around 160-180 base pairs, approximately the length of DNA wrapped around a nucleosome plus its linker. Genomic polynucleotides of a CFNA standard disclosed herein may have a size distribution similar to that of *in vivo* cfDNA. In some embodiments, at least a subset of the genomic polynucleotides have a length ranging from about 100-300 bases. For example, at least 30% (e.g., at least 35%, 40%, 45%, 50%, 55%, 60%, 65%, 70%, 75%, 80%, 85%, 90%, 95% or greater than 95%) of the genomic polynucleotides of a standard disclosed herein have a length ranging from about 100-300 bases. In some embodiments, a majority of the genomic polynucleotides of a standard disclosed herein have a length of about 100-300 bases. For example, at least 50% (e.g., at least 55%, 60%, 65%, 70%, 75%, 80%, 85%, 90%, 95% or greater than 95%) of the genomic polynucleotides of a standard disclosed herein have a length ranging from about 100-300 bases.

In some embodiments, a majority of the genomic polynucleotides of the CFNA standard are ligatable or are ready to be ligated without polynucleotide end repair and/or in the absence of a phosphate donor. Nucleic acids lacking a phosphate group at a 5' terminal end and/or a hydroxyl group at a 3' terminal end may ligate at low efficiencies or, in some cases, may not be ligated, either intermolecularly or intramolecularly. To increase the efficiency of ligation, the ends of nucleic acids lacking a phosphate group at a 5' terminal end and/or a hydroxyl group at a 3' terminal end may be repaired prior to or during ligation, for example by using an enzyme such as a polynucleotide kinase. A polynucleotide kinase may generate a ligatable polynucleotide having a phosphate group at a 5' terminal end in the presence of a phosphate donor. Non-limiting examples of end repair processes include phosphorylation of a 5' terminal nucleotide, de-phosphorylation of a 3' terminal nucleotide, polymerization with a polymerase having a 3' to 5' exonuclease activity, or a combination of these to generate a ligatable polynucleotide.

Genomic polynucleotides of CFNA standards, which are per se not claimed, disclosed herein are ligatable or are ready to be ligated without the need for such end repair. In some embodiments, at least 50% (e.g., at least 55%, 60%, 65%, 70%, 75%, 80%, 85%, 90%, 95% or greater than 95%) of the genomic polynucleotides comprise a phosphate group at the 5' terminal end and a hydroxyl group at the 3' terminal end. In some embodiments, the genomic polynucleotides of the CFNA standard are ligatable with an efficiency of at least 50% (e.g., at least 55%, 60%, 65%, 70%, 75%, 80%, 85%, 90%, 95% or greater than 95%) without end repair. In some embodiments, the genomic polynucleotides of the CFNA standard are circularized (e.g., intramolecularly ligated) with an efficiency of at least 50% (e.g., at least 55%, 60%, 65%, 70%, 75%, 80%, 85%, 90%, 95% or greater than 95%). In some embodiments, the genomic polynucleotides of the CFNA standard are ligated to adaptor polynucleotides (e.g., intermolecularly ligated) with an efficiency of at least 50% (e.g., at least 55%, 60%, 65%, 70%, 75%, 80%, 85%, 90%, 95% or greater than 95%). Where desired, end-repair is performed on genomic polynucleotides of a CFNA standard disclosed herein and/or ligating genomic polynucleotides of a CFNA standard disclosed herein (e.g., intramolecularly and/or intermolecularly) is performed in the presence of a phosphate donor. Where desired, A-tailing (e.g., the addition of an 'A' base to the 3' end of a polynucleotide) is performed on genomic polynucleotides of a CFNA standard disclosed herein, for example prior to TA cloning. In some embodiments, the genomic polynucleotides of a CFNA standard are ligated to adaptor polynucleotides (e.g., intermolecularly ligated) after A-tailing with an efficiency of at least 50% (e.g., at least 55%, 60%, 65%, 70%, 75%, 80%, 85%, 90%, 95% or greater than 95%). In some embodiments, ligation efficiency is ascertained by a polymerase chain reaction (PCR) assay, such as a quantitative PCR assay.

In some embodiments, the genomic polynucleotides are single-stranded polynucleotides. In some embodiments, the genomic polynucleotides are double-stranded polynucleotides. Double-stranded polynucleotides, in some cases, are denatured to generate single-stranded polynucleotides. In some embodiments, single-stranded genomic polynucleotides are circularized (e.g., intramolecularly ligated) with an efficiency of at least 50% (e.g., at least 55%, 60%, 65%, 70%, 75%, 80%, 85%, 90%, 95% or greater than 95%). In some embodiments, double-stranded genomic polynucleotides are circularized (e.g., intramolecularly ligated) with an efficiency of at least 50% (e.g., at least 55%, 60%, 65%, 70%, 75%, 80%, 85%, 90%, 95% or greater than 95%). In some embodiments, single-stranded genomic polynucleotides of the CFNA standard are ligated to single-stranded adaptor polynucleotides with an efficiency of at least 50% (e.g., at least 55%, 60%, 65%, 70%, 75%, 80%, 85%, 90%, 95% or greater than 95%). In some embodiments, double-stranded genomic polynucleotides of the CFNA standard are ligated to double-stranded adaptor polynucleotides with an efficiency of at least 50% (e.g., at least 55%, 60%, 65%, 70%, 75%, 80%, 85%, 90%, 95% or greater than 95%). In some embodiments, ligation efficiency is ascertained by a polymerase chain reaction (PCR) assay, such as a quantitative PCR assay.

In an aspect, the disclosure provides a method, which is not claimed, of generating a cell-free nucleic acid (CFNA) standard comprising a plurality of genomic polynucleotides. In some embodiments, the method of generating a CFNA standard comprises (a) contacting chromatin of nuclei isolated from cells to an endonuclease to generate a plurality of genomic polynucleotides comprising nucleosomal DNA fragments, and (b) optionally selecting a subset of the genomic polynucleotides, for example according to size. In some embodiments, the endonuclease cleaves DNA at regions between nucleosomes. At least a subset of the plurality of genomic polynucleotides generated by contacting chromatin of nuclei isolated from cells to an endonuclease may have a length ranging from about 100-300 bases. A majority of genomic polynucleotides generated by contacting chromatin isolated from cells to an endonuclease may have a phosphate group at 5' terminal end and hydroxyl group at a 3' terminal end.

Genomic polynucleotides of the standard may be associated with genomic sequences. In some embodiments, a standard comprises at least one subset of genomic polynucleotides (e.g., at least 2, 3, 4, 5, 6, 7, 8, 9, or 10 subsets). A CFNA standard provided herein can comprise at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 subsets. In some embodiments, a subset of genomic polynucleotides comprises members which are identical or non-unique, e.g., a given member of the subset cannot be distinguished from all other members of the subset based on nucleotide sequence and length. In some embodiments, a subset of identical members comprises at least 2, 3, 4, 5, 6, 7, 8, 9, 10, 11, 12, 13, 14, 15, 16, 17, 18, 19, 20, 21, 22, 23, 24, 25, 50, 75, or 100 identical members. The number of members in a subset of genomic polynucleotides having identical or non-unique members can depend on various factors, including, but not limited to the number of cells from which the nuclei were isolated, the types of cells from which the nuclei were obtained, and the endonuclease used to generate the plurality of genomic polynucleotides. In some embodiments, a subset of genomic polynucleotides of the standard having identical members represents less than 75% of the standard (e.g., less than 70%, 65%, 60%, 55%, 50%, 45%, 40%, 35%, 30%, 25%, 20%, 15%, 10%, or 5% of the standard). In some embodiments, a subset of genomic polynucleotides of the standard having identical members represents less than 100% of the standard. In some embodiments, a subset of genomic polynucleotides of the standard having identical members represents less than 90% of the standard. In some embodiments, a subset of genomic polynucleotides of the standard having identical members represents less than 80% of the standard. In some embodiments, a subset of genomic polynucleotides of the standard having identical members represents less than 70% of the standard. In some embodiments, a subset of genomic polynucleotides of the standard having identical members represents less than 60% of the standard. In some embodiments, a subset of genomic polynucleotides of the standard having identical members represents less than 50% of the standard. In some embodiments, a subset of genomic polynucleotides of the standard having identical members represents less than 40% of the standard. In some embodiments, a subset of genomic polynucleotides of the standard having identical members represents less than 30% of the standard. In some embodiments, a subset of genomic polynucleotides of the standard having identical members represents less than 20% of the standard. In some embodiments, a subset of genomic polynucleotides of the standard having identical members represents less than 10% of the standard. In some embodiments, less than 50% of individual genomic polynucleotides (e.g., less than 45%, 40%, 35%, 30%, 25%, 20%, 15%, 10%, 5% of individual genomic polynucleotides or less) have identical sequences.

Nuclei can be isolated from any population of cells. To obtain genomic polynucleotides having nucleotide sequences representative of a normal or non-diseased cell, nuclei may be isolated from a population of normal cells. To obtain genomic polynucleotides having nucleotide sequences representative of a diseased or abnormal cell, nuclei may be isolated from a population of abnormal or diseased cells, such as cancer cells. Genomic polynucleotides of a diseased cell may comprise, for example, one or more mutant or non-wild type alleles representative and/or associated with a disease condition. To obtain genomic polynucleotides having nucleotide sequences representative of fetal cells, nuclei may be isolated from a population of fetal cells.

Nuclei can be isolated from cells using various cell lysis reagents. Exemplary permeabilization agents for cell lysis include, but are not limited to, Triton X-100, Tween-20, saponin, SDS, NP40, streptolysin O, proteinase K, pronase and triethanolamine, and organic solvents, such as methanol and acetone. A cell sample can also be permeabilized using hypotonic shock and/or ultrasonication. Genomic DNA may remain associated with histone proteins as nucleosomal DNA and chromatin when nuclei are intact. Harvested nuclei can be treated with an enzyme such an endonuclease to cleave nucleosomal DNA (e.g., genomic DNA) into mononucleosomal-length DNA fragments. In some embodiments, the enzyme, such as an endonuclease, cleaves DNA at regions between nucleosomes. Nucleosomal DNA isolated from cells or isolated nuclei may, in some cases, be treated with an enzyme such as an endonuclease to cleave the nucleosomal DNA (e.g., genomic DNA) into mononucleosomal-length DNA fragments. Nucleosomal DNA may be harvested without an intermediate step of harvesting nuclei. In some embodiments, a majority of fragments generated by cleaving nucleosomal DNA with an endonuclease comprises a phosphate group at a 5' terminal end and a hydroxyl group at a 3' terminal end and are ligatable or are ready to be ligated without end repair. In some embodiments, at least 50% (e.g., at least 55%, 60%, 65%, 70%, 75%, 80%, 85%, 90%, 95% or greater than 95%) of the genomic polynucleotides generated by cleaving nucleosomal DNA with an endonuclease comprise a phosphate group at the 5' terminal end and a hydroxyl group at the 3' terminal end. In some embodiments, the genomic polynucleotides of the CFNA standard are ligatable with an efficiency of at least 50% (e.g., at least 55%, 60%, 65%, 70%, 75%, 80%, 85%, 90%, 95% or greater than 95%) without end repair and/or in the absence of a phosphate donor. Examples of endonucleases for generating genomic polynucleotides include, but are not limited to, caspase-activated DNase (CAD), DNase I including single-strand specific and double-strand specific DNases, DNase γ, and Endonuclease G. In some embodiments, the genomic polynucleotides are not generated by sonication, for example by sonicating genomic DNA to generate genomic polynucleotides. In some embodiments, the genomic polynucleotides are not generated by contacting nucleosomal DNA to micrococcal nuclease.

The genomic polynucleotides may be purified prior to use, for example use in a CFNA assay. Genomic polynucleotides may be subjected to size exclusion chromatography, whereby small reagents are retained and discarded, or genomic polynucleotides are retained and released in a separate volume. Kits for nucleosomal DNA purification are available, such as kits provided by Zymo Research. Genomic polynucleotides may be optionally selected for size (e.g., length) prior to use, for example to enrich the proportion of genomic polynucleotides that are mononucleosomal in length.

CFNA standards, which are per se not claimed, disclosed herein may comprise a mixture of nucleic acids that are representative of certain *in vivo* CFNA samples, for example samples comprising a mixture of nucleic acids from normal, healthy cells of a subject and nucleic acids derived from tumor cells, nucleic acids derived from fetal cells, nucleic acids derived from a non-autologous source (e.g., cell or tissue transplant), and/or a pathogen (e.g., bacteria or virus). CFNA standards representative of *in vivo* CFNA samples comprising a mixture of nucleic acids can be generated by spiking-in genomic polynucleotides, for example, from tumor cells or fetal cells (e.g., reference nucleic acid or reference polynucleotide) into a sample comprising genomic polynucleotides of healthy cells. The genomic polynucleotides of healthy cells and diseased or fetal cells can be mixed together in specific ratios to generate a CFNA standard comprising a reference polynucleotide present in the standard at a desired amount (e.g., expected abundance).

In some embodiments of such standards, a nucleic acid comprising a mutant allele from a tumor or a fetal nucleic acid as a reference polynucleotide is used to develop a CFNA assay, validate a CFNA assay, optimize a CFNA assay, and/or evaluate the performance of a CFNA assay. In some embodiments, detection of the reference polynucleotide is used to validate, verify, and/or normalize the results obtained from a CFNA assay. In some embodiments, a CFNA assay may be evaluated for its ability to detect the presence of the reference polynucleotide in a cell sample. This ability to detect the presence of a reference polynucleotide in a CFNA standard may be representative of the assay's ability to detect a target nucleic acid in a cell-free nucleic acid sample.

In an aspect, the disclosure provides a method for estimating abundance of a target nucleic acid present in a cell-free nucleic acid (CFNA) sample, as defined in the appended claims, comprising the target nucleic acid and non-target nucleic acids. In some embodiments, the method comprises (a) quantifying the copy number of the target nucleic acid in the CFNA sample to obtain an observed abundance of the target nucleic acid; (b) generating a calibration scheme by correlating an observed abundance of a reference nucleic acid present in a CFNA standard to an expected abundance of the reference nucleic acid present in the CFNA standard, which CFNA standard comprises a plurality of genomic polynucleotides, individual members of the plurality having a 5' terminal end and a 3' terminal end, wherein: (i) at least a subset of the plurality of genomic polynucleotides have a length ranging from about 100-300 bases; and (ii) a majority of the genomic polynucleotides have a phosphate group at a 5' terminal end and a hydroxyl group at a 3' terminal end, which majority of genomic polynucleotides are ligatable without generating a phosphate group at a 5' terminal end and/or generating a hydroxyl group at a 3' terminal end; and (c) estimating abundance of the target nucleic acid in the CFNA sample by adjusting the observed abundance of the target nucleic acid using the calibration scheme.

A CFNA sample may comprise cell-free nucleic acids (e.g., cfDNA and/or cfRNA) obtained from a subject, for example from a biological sample of the subject. In some embodiments, the subject is healthy, and cell-free nucleic acids obtained from the subject may not comprise a sequence variant associated with a disease or disorder. In some embodiments, the subject is suspected of having a disease or disorder, and cell-free nucleic acids obtained from the subject may comprise a sequence variant associated with the disease or disorder. In some embodiments, the subject is pregnant, and cell-free nucleic acids obtained from the subject comprise fetal nucleic acids. In some embodiments, the subject is a patient that has undergone a transplant, and cell-free nucleic acids obtained from the subject comprise cell-free nucleic acids from the non-autologous cells or tissue. In some embodiments, the subject is suspected of being infected by a pathogen such as a bacteria, virus, or fungus, and cell-free nucleic acids obtained from the subject comprise nucleic acids derived from the pathogen.

A target nucleic acid of the sample may refer to any nucleic acid that is desired to be detected. For example, in a sample obtained from a subject suspected of having a disease such as a cancer or tumor, the target nucleic acid may refer to a mutant allele known to be associated (e.g., weakly, moderately, or strongly) with the suspected cancer or tumor. The presence of the mutant allele in a sample may be indicative of the presence of the disease (e.g., cancer) in the subject. Therefore, by detecting the presence of the mutant allele, the presence of the disease (e.g., cancer), in some cases, may be inferred. Where the target nucleic acid is a mutant allele, non-target nucleic acids may refer to the wild-type allele. In some cases, non-target nucleic acids may refer to the entire population of nucleic acids of the sample that are not the target nucleic acid.

The CFNA sample can be analyzed by various methods to quantify the copy number of the target nucleic acid in the sample (e.g., the number of target nucleic acid molecules in the sample) to yield an observed abundance of the target nucleic acid. An observed abundance refers to the abundance that is detected in the sample, for example using a particular assay or method. In some cases, an observed abundance of a target nucleic acid in a CFNA sample as detected by a first method or assay may be different from an observed abundance of the same target nucleic acid in the same CFNA sample as detected by a second method or assay due to differences between the two methods or assay, such as differences in efficiency of sample recovery in various sample manipulation stages (e.g., nucleic acid extraction, purification, amplification, ligation, digestion, etc) and/or error associated with each stage.

Observed abundance may be described as a fraction or percentage, for example of the total (e.g., total number of target polynucleotide molecules observed relative to total number of polynucleotide molecules observed in entire sample) or a subset of the total (e.g., total number of molecules of a mutant allele observed relative to the total number of molecules of the gene observed, including mutant and wild-type alleles). Abundance may, in some cases, be described as a concentration including, but not limited to, mass concentration, molar concentration, number concentration, and volume concentration, or other acceptable unit of measure. Where a target nucleic acid is a mutant allele, the observed abundance may be expressed as an allelic frequency of the mutant allele.

In some embodiments, the observed abundance of the target nucleic acid is determined by an amplification reaction, such as digital polymerase chain reaction (dPCR), droplet digital polymerase chain reaction (ddPCR), or quantitative polymerase chain reaction (qPCR). An amplification reaction (e.g., dPCR, ddPCR, or qPCR) may be performed with amplification primers specific to the target nucleic acid. For example, primers may be allele specific primers if a target nucleic acid is a mutant allele.

In some embodiments, the observed abundance of the target nucleic acid present in the CFNA sample is determined by (a) sequencing a plurality of amplification products to generate a plurality of sequence reads, wherein the plurality of amplification products are generated by amplifying the target nucleic acid and non-target nucleic acids of the CFNA sample; and (b) analyzing the sequence reads to calculate the observed abundance of the target nucleic acid.

Amplifying target nucleic acids and non-target nucleic acids may be used to increase the amount of material available for analysis, for example sequencing analysis, if the amount of starting material is low and/or insufficient to, e.g., assess copy number of the target nucleic acid. In some embodiments, the nucleic acid sample comprising target nucleic acid and non-target nucleic acids are circularized to produce a plurality of circularized target nucleic acids and a plurality of circularized non-target nucleic acids prior to amplification. In some embodiments, circularizing is effected by subjecting the target nucleic acid and the non-target nucleic acids present in the CFNA sample to a ligation reaction. Ligation may be accomplished, for example, by a ligase enzyme. Nucleic acids having a phosphate group at a 5' terminal end and a hydroxyl group at a 3' terminal end can be circularized in the absence of a phosphate donor and/or without a preceding end repair step (e.g., using a polynucleotide kinase enzyme). In some embodiments, the target nucleic acid and the non-target nucleic acids are circularized (e.g., intramolecularly ligated) with an efficiency of at least 50% (e.g., at least 55%, 60%, 65%, 70%, 75%, 80%, 85%, 90%, 95% or greater than 95%). In some embodiments, single-stranded target nucleic acid and single-stranded non-target nucleic acids are circularized (e.g., intramolecularly ligated) with an efficiency of at least 50% (e.g., at least 55%, 60%, 65%, 70%, 75%, 80%, 85%, 90%, 95% or greater than 95%). In some embodiments, double-stranded target nucleic acid and double-stranded non-target nucleic acids are circularized with an efficiency of at least 50% (e.g., at least 55%, 60%, 65%, 70%, 75%, 80%, 85%, 90%, 95% or greater than 95%). Where desired, end-repair is performed on the target nucleic acid and non-target nucleic acids of the nucleic acid sample and/or ligating the target nucleic acid and non-target nucleic acids of the nucleic acid sample (e.g., intramolecularly and/or intermolecularly) is performed in the presence of a phosphate donor. Ligation efficiency can be ascertained by a polymerase chain reaction (PCR) assay, such as a quantitative PCR assay. In some embodiments, uncircularized target nucleic acid and uncircularized non-target nucleic acids are removed, for example by degradation or digestion, prior to amplification.

Amplifying circularized target nucleic acids and circularized non-target nucleic acids, in some embodiments, comprises isothermal amplification such as rolling circle amplification. In some embodiments, amplifying by rolling circle amplification comprises extension of random primers. Random primers having a random nucleotide sequence can hybridize and prime various random regions of a circular target polynucleotide. In some embodiments, amplifying comprises extension of one or more primers specific to a target sequence, for example that of a target nucleic acid. In some embodiments, the primers used in primer extension may comprise at least one sequence element selected from amplification primer binding site, a sequencing primer binding site, a barcode sequence, a sample index sequence, a sequence to bind a polynucleotide to a flow cell for next generation sequence, and/or a restriction enzyme sequence. Sequence elements may facilitate further downstream analysis. For example, sequencing primer binding sequences may be useful for sequencing the amplification products.

In some embodiments, the target nucleic acid and the non-target nucleic acids are joined to adaptor polynucleotides to produce a plurality of adaptor-tagged target nucleic acids and a plurality of adaptor-tagged non-target nucleic acids prior to amplification. Adaptor polynucleotides generally refer to oligonucleotides incorporated at the 5' and/or 3' ends of polynucleotides to facilitate one or more downstream analysis steps, for example in an amplification and/or a sequencing reaction. For example, an adaptor polynucleotide may contain one or more of a variety of sequence elements including, but not limited to, an amplification primer binding site, a sequencing primer binding site, a barcode sequence, a sample index sequence, a sequence to bind a polynucleotide to a flow cell for next generation sequence, and/or a restriction enzyme sequence. In some embodiments, joining to adaptor polynucleotides is effected by subjecting the target nucleic acid and the non-target nucleic acids to a ligation reaction. Ligation can be accomplished, for example, by a ligase enzyme.

Nucleic acids having a phosphate group at a 5' terminal end and a hydroxyl group at a 3' terminal end can be ligated to adaptor polynucleotides in the absence of a phosphate donor and/or without a preceding end repair step (e.g., using a polynucleotide kinase enzyme). In some embodiments, the target nucleic acid and the non-target nucleic acids are ligated to adaptor polynucleotides with an efficiency of at least 50% (e.g., at least 55%, 60%, 65%, 70%, 75%, 80%, 85%, 90%, 95% or greater than 95%). In some embodiments, single-stranded target nucleic acid and single-stranded non-target nucleic acids are ligated to single-stranded adaptor polynucleotides with an efficiency of at least 50% (e.g., at least 55%, 60%, 65%, 70%, 75%, 80%, 85%, 90%, 95% or greater than 95%). In some embodiments, double-stranded target nucleic acid and double-stranded non-target nucleic acids are ligated to double-stranded adaptor polynucleotides with an efficiency of at least 50% (e.g., at least 55%, 60%, 65%, 70%, 75%, 80%, 85%, 90%, 95% or greater than 95%). Where desired, end-repair is performed on the target nucleic acid and non-target nucleic acids of the nucleic acid sample and/or ligating the target nucleic acid and non-target nucleic acids of the nucleic acid sample (e.g., intramolecularly and/or intermolecularly) is performed in the presence of a phosphate donor. Where desired, A-tailing (e.g., the addition of an 'A' base to the 3' end of a polynucleotide) is performed on genomic polynucleotides of a CFNA standard disclosed herein, for example prior to TA cloning. In some embodiments, the genomic polynucleotides of a CFNA standard are ligated to adaptor polynucleotides (e.g., intermolecularly ligated) after A-tailing with an efficiency of at least 50% (e.g., at least 55%, 60%, 65%, 70%, 75%, 80%, 85%, 90%, 95% or greater than 95%). In some embodiments, ligation efficiency is ascertained by a polymerase chain reaction (PCR) assay, such as a quantitative PCR assay.

Amplifying the adaptor-tagged nucleic acids, in some embodiments, comprises extension of one or more primers specific to a primer binding sequence of the adaptor polynucleotide. Amplification may comprise either an isothermal amplification reaction or a thermocycling amplification reaction.

A CFNA standars, which is per se not claimed, comprising genomic polynucleotides can be assayed to generate a calibration scheme. A calibration scheme can be generated by correlating an observed abundance of a reference nucleic acid in the standard with the expected abundance of the reference nucleic acid. In some embodiments, generating the calibration scheme further comprises correlating an observed abundance of the reference nucleic acid present in an additional CFNA standard to an expected abundance of the reference nucleic acid present in the additional CFNA standard. In some embodiments, the expected abundance of the reference nucleic acid present in the additional CFNA standard is different from the expected abundance of the reference nucleic acid present in the CFNA standard. Using two different standards having two different expected abundances of the reference nucleic acid generates two data points (e.g., calibration data points) that can be used for generating the calibration scheme. In some embodiments, at least 3, 4, 5, 6, 7, 8, 9, 10 or greater than 10 CFNA standards, each standard having the same reference nucleic acid present at different expected abundances, is used to generate the calibration scheme.

In some embodiments, the calibration scheme comprises a calibration algorithm that is used for adjusting the observed abundance of the target nucleic acid to yield an estimated abundance of the target nucleic acid, for example correcting for deviation of an observed abundance of the reference nucleic acid from an expected abundance of the reference nucleic acid for a plurality of expected abundance levels of the reference nucleic acid. In some embodiments, the calibration scheme comprises a line of best fit, for example a line of best fit for a plurality of calibration data points.

The expected abundance of the reference nucleic acid present in the CFNA standard may be selected based on hypothesized or verified characteristics or properties of the CFNA sample and/or assay system. For example, a CFNA standard chosen for a CFNA assay may comprise a reference nucleic acid at an expected abundance that most closely resembles a predicted abundance of the target nucleic acid in the sample. A predicted abundance refers to an abundance that is hypothesized based on certain characteristics of a sample but is not necessarily expected to be or believed to be true. A CFNA standard chosen for a CFNA assay may comprise a reference nucleic acid at an expected abundance that, alternatively, has been optimized for use with a particular method or assay rather than the CFNA sample. In some embodiments, the expected abundance of the reference nucleic acid present in CFNA standard is less than 20% (e.g., less than 10%, 5%, 1%, 0.5%, 0.1%, 0.01%, 0.001% or less than 0.001%). In some embodiments, the reference nucleic acid comprises a mutant allele. Where the reference nucleic acid comprises a mutant allele, the expected abundance may be expressed as an allelic frequency. In some embodiments, the mutant allele is present in the CFNA standard at an allelic frequency of about 50% (e.g., representative of a germline heterozygous mutation). In some embodiments, the mutant allele is present in the CFNA standard at an allelic frequency of less than 50% (e.g., less than 40%, 30%, 20%, 10%, 5%, 1%, 0.5%, 0.1%, 0.01%, 0.001% or less than 0.001%).

In some embodiments, a CFNA standard, which is per se not claimed, comprises multiple reference nucleic acids (e.g., at least one additional reference nucleic acid) in a single CFNA standard. The presence of multiple reference nucleic acids may allow for multiplex analysis. The multiple reference nucleic acids may be independently identifiable (e.g., by amplification and/or sequencing) in the single CFNA standard. Each of the multiple reference nucleic acids may be present at about the same expected abundance. In some cases, each of the multiple reference nucleic acids is present at an expected abundance different from each other reference of the standard. In some embodiments, generating the calibration scheme further comprises correlating an observed abundance of at least one additional reference nucleic acid present in the single CFNA standard to an expected abundance of the at least one additional reference nucleic acid present in the single CFNA standard. In some embodiments, at least 3, 4, 5, 6, 7, 8, 9, 10 or greater than 10 unique reference nucleic acids in a single CFNA standard are used to generate a calibration scheme, each standard present at about the same expected abundance. In some embodiments, the expected abundance of the at least one additional reference nucleic acid present in single CFNA standard is less than 20% (e.g., less than 10%, 5%, 1%, 0.5%, 0.1%, 0.01%, 0.001% or less than 0.001%). In some embodiments, the at least one additional reference nucleic acid comprises an additional mutant allele. Where the at least one additional reference nucleic acid comprises a mutant allele, the expected abundance of the additional reference nucleic acid may be expressed as an allelic frequency. In some embodiments, the additional mutant allele is present in the single CFNA standard at an allelic frequency of about 50% (e.g., representative of a germline heterozygous mutation). In some embodiments, the additional mutant allele is present in the single CFNA standard at an allelic frequency of less than 50% (e.g., less than 40%, 30%, 20%,10%, 5%, 1%, 0.5%, 0.1%, 0.01%, 0.001% or less than 0.001%).

Genomic polynucleotides of a CFNA standard in accordance with an embodiment may have lengths reflective of the size distribution of *in vivo* CFNA. In some embodiments, at least a subset of the genomic polynucleotides have a length ranging from about 100-300 bases. For example, at least 30% (e.g, at least 35%, 40%, 45%, 50%, 55%, 60%, 65%, 70%, 75%, 80%, 85%, 90%, 95% or greater than 95%) of the genomic polynucleotides have a length ranging from about 100-300 bases. In some embodiments, a majority of the genomic polynucleotides have a length ranging from about 100-300 bases. For example, at least 50% (e.g., at least 55%, 60%, 65%, 70%, 75%, 80%, 85%, 90%, 95% or greater than 95%) of the genomic polynucleotides have a length ranging from about 100-300 bases.

In some embodiments, a standard, which is per se not claimed, comprises at least one subset of genomic polynucleotides (e.g., at least 2, 3, 4, 5, 6, 7, 8, 9, or 10 subsets). In some embodiments, a CFNA standard provided herein comprises at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 subsets. The subset of genomic polynucleotides can comprise members which are identical or non-unique, e.g., a given member of the subset cannot be distinguished from all other members of the subset based on nucleotide sequence and length. In some embodiments, a subset of identical members comprises at least 2, 3, 4, 5, 6, 7, 8, 9, 10, 11, 12, 13, 14, 15, 16, 17, 18, 19, 20, 21, 22, 23, 24, 25, 50, 75, or 100 identical members. In some embodiments, a subset of genomic polynucleotides of the standard having identical members represents less than 75% of the standard (e.g., less than 70%, 65%, 60%, 55%, 50%, 45%, 40%, 35%, 30%, 25%, 20%, 15%, 10%, or 5% of the standard). In some embodiments, a subset of genomic polynucleotides of the standard having identical members represents less than 100% of the standard. In some embodiments, a subset of genomic polynucleotides of the standard having identical members represents less than 90% of the standard. In some embodiments, a subset of genomic polynucleotides of the standard having identical members represents less than 80% of the standard. In some embodiments, a subset of genomic polynucleotides of the standard having identical members represents less than 70% of the standard. In some embodiments, a subset of genomic polynucleotides of the standard having identical members represents less than 60% of the standard. In some embodiments, a subset of genomic polynucleotides of the standard having identical members represents less than 50% of the standard. In some embodiments, a subset of genomic polynucleotides of the standard having identical members represents less than 40% of the standard. In some embodiments, a subset of genomic polynucleotides of the standard having identical members represents less than 30% of the standard. In some embodiments, a subset of genomic polynucleotides of the standard having identical members represents less than 20% of the standard. In some embodiments, a subset of genomic polynucleotides of the standard having identical members represents less than 10% of the standard. In some embodiments, less than 50% of individual genomic polynucleotides (e.g., less than 45%, 40%, 35%, 30%, 25%, 20%, 15%, 10%, 5% or less than 5% of the genomic polynucleotides) of the CFNA standard have identical sequences.

In some embodiments, the CFNA standard comprises single-stranded genomic polynucleotides. In some embodiments, the CFNA standard comprises double-stranded genomic polynucleotides. Double-stranded genomic polynucleotides can be treated, for example by denaturation, to yield single-stranded polynucleotides if desired.

An observed abundance of the reference polynucleotide in the CFNA standard may be quantified by the same method as that used to obtain the observed abundance of the target nucleic acid in the CFNA sample. For example, the observed abundance of the reference nucleic acid in the CFNA sample may be determined by an amplification reaction such as dPCR, ddPR, or qPCR. Alternatively, the observed abundance of the reference nucleic acid in the CFNA sample may be determined by sequencing a plurality of amplification products to generate a plurality of sequence reads, the plurality of amplification products generated by amplifying the CFNA standard. In some embodiments, nucleic acids of the CFNA standard are circularized prior to amplification, for example by intramolecular ligation. In some embodiments, nucleic acids of the CFNA standard are joined to adaptor polynucleotides prior to amplification, for example by intermolecular ligation. Ligation (e.g., intramolecular and/or intermolecular) may be accomplished, for example, by a ligase enzyme.

Nucleic acids having a phosphate group at a 5' terminal end and a hydroxyl group at a 3' terminal end can be circularized and/or ligated to adaptor polynucleotides in the absence of a phosphate donor and/or without a preceding end repair step (e.g., using a polynucleotide kinase enzyme). For example, genomic polynucleotides of the CFNA standard having a phosphate group at a 5' terminal end and a hydroxyl group at a 3' terminal end are ligatable or are ready to be ligated without generating a phosphate group at a 5' terminal end and/or generating a hydroxyl group at a 3' terminal end. In some embodiments, at least 50% (e.g., at least 55%, 60%, 65%, 70%, 75%, 80%, 85%, 90%, 95% or greater than 95%) of the genomic polynucleotides comprise a phosphate group at the 5' terminal end and a hydroxyl group at the 3' end. In some embodiments, the genomic polynucleotides of the CFNA standard are ligatable with an efficiency of at least 50% (e.g., at least 55%, 60%, 65%, 70%, 75%, 80%, 85%, 90%, 95% or greater than 95%). In some embodiments, the genomic polynucleotides of the CFNA standard are circularized (e.g., intramolecularly ligated) with an efficiency of at least 50% (e.g., at least 55%, 60%, 65%, 70%, 75%, 80%, 85%, 90%, 95% or greater than 95%). In some embodiments, single-stranded genomic polynucleotides are circularized (e.g., intramolecularly ligated) with an efficiency of at least 50% (e.g., at least 55%, 60%, 65%, 70%, 75%, 80%, 85%, 90%, 95% or greater than 95%). In some embodiments, double-stranded genomic polynucleotides are circularized (e.g., intramolecularly ligated) with an efficiency of at least 50% (e.g., at least 55%, 60%, 65%, 70%, 75%, 80%, 85%, 90%, 95% or greater than 95%). In some embodiments, the genomic polynucleotides of the CFNA standard are ligated to adaptor polynucleotides with an efficiency of at least 50% (e.g., at least 55%, 60%, 65%, 70%, 75%, 80%, 85%, 90%, 95% or greater than 95%). In some embodiments, single-stranded genomic polynucleotides of the CFNA standard are ligated to single-stranded adaptor polynucleotides with an efficiency of at least 50% (e.g., at least 55%, 60%, 65%, 70%, 75%, 80%, 85%, 90%, 95% or greater than 95%). In some embodiments, double-stranded genomic polynucleotides of the CFNA standard are ligated to double-stranded adaptor polynucleotides with an efficiency of at least 50% (e.g., at least 55%, 60%, 65%, 70%, 75%, 80%, 85%, 90%, 95% or greater than 95%). In some embodiments, ligation efficiency is ascertained by a polymerase chain reaction (PCR) assay, such as a quantitative PCR assay.

In some embodiments, a phosphate group at a 5' terminal end and a hydroxyl group at a 3' terminal end of a genomic polynucleotide are not generated by a polynucleotide kinase, for example in an end repair process. That is, genomic polynucleotides of the CFNA standard are ligatable or are ready to be ligated without generating a phosphate group at a 5' terminal end and/or generating a hydroxyl group at a 3' terminal end, such as generation in a repair process that is in addition to an initial process that produces individual members of a standard from genomic DNA. In some embodiments, the genomic polynucleotides of the CFNA standard are ligatable or are ready to be ligated in the absence of a phosphate donor. Where desired, end-repair is performed on genomic polynucleotides of the CFNA standard and/or ligating genomic polynucleotides of the CFNA standard is performed in the presence of a phosphate donor.

In some embodiments, the nucleotide sequence of the reference nucleic acid is at least 85% identical (e.g., at least 90%, 95%, or greater than 95% identical) to the target nucleic acid.

In an aspect, the disclosure provides a method, which is not claimed, for assessing a detection limit of a cell-free nucleic acid (CFNA) assay. In some embodiments, the method comprises (a) performing the CFNA assay with a plurality of CFNA standards to obtain for each standard an observed abundance of a reference polynucleotide present in each standard, the plurality of CFNA standards covering a given range of expected abundances of the reference polynucleotide, wherein each standard of the plurality has an expected abundance of the reference polynucleotide that is different from that of other standards of the plurality, and wherein each standard comprises a plurality of genomic polynucleotides, individual members of the plurality having a 5' terminal end and a 3' terminal end, wherein: (i) at least a subset of the plurality of genomic polynucleotides have a length ranging from about 100-300 bases; and (ii) a majority of the genomic polynucleotides have a phosphate group at a 5' terminal end and a hydroxyl group at a 3' terminal end, which majority of genomic polynucleotides are ligatable without generating a phosphate group at a 5' terminal end and/or generating a hydroxyl group at a 3' terminal end; and (c) identifying an expected abundance at which a corresponding observed abundance of the reference polynucleotide is statistically indistinguishable from a background measurement, thereby calling the expected abundance as the detection limit of the CFNA assay.

A CFNA standard, which is per se not claimed, disclosed herein may be useful in determining the detection limit of an assay, for example a CFNA assay involving an instrument (e.g., a dPCR machine, a ddPCR machine, a qPCR machine, an NGS machine, etc) or specific method (e.g., a method including steps of nucleic acid extraction, purification, ligation, amplification, digestion, etc). The assay may be performed, for example, with a plurality of CFNA standards, each of which has a reference polynucleotide at an expected abundance and, collectively, the expected abundances of the reference polynucleotides of the standards span a given range. In some embodiments, the plurality of CFNA standards comprises at least 2 CFNA standards (e.g., at least 3, 4, 5, 6, 7, 8, 9, 10 or greater than 10 CFNA standards). For each standard, the observed abundance of the reference polynucleotide may be compared to an observed abundance of a sample containing no nucleic acid (e.g., a background measurement or noise level) to determine the expected abundance at which the observed abundance of the reference polynucleotide from the standards is indistinguishable from the background measurement.

A CFNA standard, which is per se not claimed, disclosed herein may be useful in determining the detection limit of an assay, for example a CFNA assay involving an instrument (e.g., a dPCR machine, a ddPCR machine, a qPCR machine, an NGS machine, etc) or specific method (e.g., a method including steps of nucleic acid extraction, purification, ligation, amplification, digestion, etc). The assay may be performed, for example, with a single CFNA standard having a plurality of unique reference polynucleotides (e.g., each of the reference polynucleotides is independently identifiable, for example by amplification and/or sequencing). A single CFNA standard may comprise at least 2 reference polynucleotides (e.g., at least 3, 4, 5, 6, 7, 8, 9, 10 or greater than 10 reference polynucleotides). Each of the reference polynucleotides present in the CFNA standard may be present in the standard at about the same expected abundance. In some embodiments, the same expected abundance is less than about 20%, 10%, 5%, 1%, 0.5%, 0.1%, 0.01%, 0.001% or less than 0.0001%. In some embodiments, each reference polynucleotide of the CFNA standard comprises a mutant allele. Where each reference polynucleotide of the CFNA standard comprises a mutant allele, the expected abundance of the reference polynucleotide may be an allelic frequency of less than about 50%, 40%, 30%, 20%, 10%, 5%, 1%, 0.5%, 0.1%, 0.01%, or 0.001%. In some embodiments, the allelic frequency of the mutant allele is less than 0.05%.

In an aspect, the disclosure provides a method, which is not claimed, for assessing sensitivity of a cell-free nucleic acid (CFNA) assay for detecting reference polynucleotides present in a CFNA standard at a given expected abundance, comprising (a) performing the CFNA assay with a CFNA standard comprising a plurality of reference polynucleotides to obtain a positive or negative detection call for each reference polynucleotide of the plurality, wherein each reference polynucleotide is present in the CFNA standard at the given expected abundance, wherein the standard comprises a plurality of genomic polynucleotides, individual members of the plurality having a 5' terminal end and a 3' terminal end, and wherein: at least a subset of the plurality of genomic polynucleotides have a length ranging from about 100-300 bases; and a majority of the genomic polynucleotides have a phosphate group at a 5' terminal end and a hydroxyl group at a 3' terminal end, which majority of genomic polynucleotides are ligatable without generating a phosphate group at a 5' terminal end and/or generating a hydroxyl group at a 3' terminal end; and (b) determining the fraction of reference polynucleotides yielding a positive detection call, thereby assessing the sensitivity of the CFNA assay for detecting reference polynucleotides present in the CFNA standard at the given expected abundance. The sensitivity of an assay refers to a measure of the assay's performance in correctly identifying or measuring positive events, for example correctly calling the presence of a reference polynucleotide in a standard. The sensitivity of a CFNA assay for detecting a predetermined or given number of reference polynucleotides in a CFNA standard can be calculated by determining the fraction of positive detection calls.

In an aspect, the disclosure provides a method, which is not claimed, for assessing specificity of a cell-free nucleic acid (CFNA) assay, comprising (a) performing the CFNA assay with a CFNA standard to obtain a positive or negative detection call for each of a plurality of reference polynucleotides, wherein each reference polynucleotide is absent in the CFNA standard, and wherein at least a subset of the plurality of genomic polynucleotides have a length ranging from about 100-300 bases; and a majority of the genomic polynucleotides have a phosphate group at a 5' terminal end and a hydroxyl group at a 3' terminal end, which majority of genomic polynucleotides are ligatable without generating a phosphate group at a 5' terminal end and/or generating a hydroxyl group at a 3' terminal end; and (b) determining the fraction of reference polynucleotides yielding a negative detection call, thereby assessing the specificity of the CFNA assay. The specificity of a CFNA assay refers to a measure of the assay's performance in correctly identifying or measuring negative events, for example correctly calling the absence of a reference polynucleotide in a standard. The specificity of a CFNA assay can be calculated by determining the fraction of negative detection calls for a plurality of reference polynucleotides that are absent in a standard.

The presence of multiple reference nucleic acids in a single CFNA standard may allow for multiplex analysis, for example in assessing the detection limit, sensitivity and/or specificity of a CFNA assay. The observed abundances of each of the reference polynucleotides of the single CFNA standard may be determined in parallel, for example by amplification (e.g., dPCR, ddPCR, qPCR) and/or sequencing, when assessing the detection limit of a CFNA assay. As each reference polynucleotide is unique from the others, each reference polynucleotide yields a data point comprising an observed abundance and corresponding expected abundance. For example, a CFNA assay may be performed on a single CFNA standard having 10 reference polynucleotides to yield observed abundance values for each of the 10 reference polynucleotides. If for at least a majority of the reference polynucleotides (e.g., at least 50%, 55%, 60%, 65%, 70% 75%, 80%, 85%, 90%, 95% or greater than 95% of the reference polynucleotides), the observed abundance is within about 20% (e.g., about 15%, 10%, 5%, 4%, 3%, 2%, 1% or less than 1%) of the expected abundance, the detection limit of the assay may be called as the same expected abundance of the reference polynucleotides. In some embodiments, the detection limit is called as the expected abundance of the reference polynucleotides when at least 75% of the reference polynucleotides present in the standard are detected at about the expected abundance. A plurality of reference polynucleotides present in a single CFNA standard allows multiple reference polynucleotides (e.g., at least 2, 3, 4, 5, 6, 7, 8, 9, 10, 20, 30, 40, 50, 60, 70, 80, 90, 100, or greater than 100 reference polynucleotides) to be analyzed under the same or similar assay conditions, for example in determining sensitivity and/or specificity of a CFNA assay, if the CFNA standard is treated a single sample. This may reduce variability in sensitivity and/or specificity measurements resulting from inter-experiment variability.

In some embodiments, the given range of expected abundances of the reference polynucleotides is from about 0.001% to a 20% (e.g., between about 0.001% to a 15%, 0.001% to a 10%, 0.001% to a 5%, 0.001% to a 4%, 0.001% to a 3%, 0.001% to a 2%, 0.001% to a 1%, 0.001% to a 0.5%, 0.001% to a 0.1%, or 0.001% to a 0.01%). In some embodiments, the reference polynucleotide comprises a mutant allele. Where the reference polynucleotide comprises a mutant allele, the expected abundance may be expressed as an allelic frequency of the mutant allele. The given range of expected abundances of the reference polynucleotide comprising a mutant allele may be an allelic frequency from about 0.001% to 50% (e.g., between about 0.001% to a 45%, 0.001% to a 40%, 0.001% to a 35%, 0.001% to a 30%, 0.001% to a 25%, 0.001% to a 20%, 0.001% to a 15%, 0.001% to a 10%, 0.001% to a 5%, 0.001% to a 4%, 0.001% to a 3%, 0.001% to a 2%, 0.001% to a 1%, 0.001% to a 0.5%, 0.001% to a 0.1%, or 0.001% to a 0.01%).

Genomic polynucleotides of a CFNA standard useful for assessing a detection limit of a CFNA assay, the sensitivity of a CFNA assay, and/or the specificity of a CFNA assay may have lengths reflective of the size distribution of *in vivo* CFNA. In some embodiments, at least a subset of the genomic polynucleotides have a length ranging from about 100-300 bases. For example, at least 30% (e.g., at least 35%, 40%, 45%, 50%, 55%, 60%, 65%, 70%, 75%, 80%, 85%, 90%, 95% or greater than 95%) of the genomic polynucleotides have a length ranging from about 100-300 bases. In some embodiments, at least a majority of genomic polynucleotides have a length ranging from about 100-300 bases. For example, at least 50% (e.g., at least 55%, 60%, 65%, 70%, 75%, 80%, 85%, 90%, 95% or greater than 95%) of the genomic polynucleotides have a length ranging from about 100-300 bases.

In some embodiments, a standard, which is per se not claimed, useful for assessing a detection limit of a CFNA assay, the sensitivity of a CFNA assay, and/or the specificity of a CFNA assay comprises at least one subset of genomic polynucleotides (e.g., at least 2, 3, 4, 5, 6, 7, 8, 9, or 10 subsets). In some embodiments, the CFNA standard comprises at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, or 500 subsets. In some embodiments, a subset of genomic polynucleotides comprises members which are identical or non-unique, e.g., a given member of the subset cannot be distinguished from all other members of the subset based on nucleotide sequence and length. In some embodiments, a subset of identical members comprises at least 2, 3, 4, 5, 6, 7, 8, 9, 10, 11, 12, 13, 14, 15, 16, 17, 18, 19, 20, 21, 22, 23, 24, 25, 50, 75, or 100 identical members. In some embodiments, a subset of genomic polynucleotides of the standard having identical members represents less than 75% of the standard (e.g., less than 70%, 65%, 60%, 55%, 50%, 45%, 40%, 35%, 30%, 25%, 20%, 15%, 10%, or 5% of the standard). In some embodiments, a subset of genomic polynucleotides of the standard having identical members represents less than 100% of the standard. In some embodiments, a subset of genomic polynucleotides of the standard having identical members represents less than 90% of the standard. In some embodiments, a subset of genomic polynucleotides of the standard having identical members represents less than 80% of the standard. In some embodiments, a subset of genomic polynucleotides of the standard having identical members represents less than 70% of the standard. In some embodiments, a subset of genomic polynucleotides of the standard having identical members represents less than 60% of the standard. In some embodiments, a subset of genomic polynucleotides of the standard having identical members represents less than 50% of the standard. In some embodiments, a subset of genomic polynucleotides of the standard having identical members represents less than 40% of the standard. In some embodiments, a subset of genomic polynucleotides of the standard having identical members represents less than 30% of the standard. In some embodiments, a subset of genomic polynucleotides of the standard having identical members represents less than 20% of the standard. In some embodiments, a subset of genomic polynucleotides of the standard having identical members represents less than 10% of the standard. In some embodiments, less than 50% of individual genomic polynucleotides (e.g., less than 45%, 40%, 35%, 30%, 25%, 20%, 15%, 10%, 5% or less than 5% of the genomic polynucleotides) of the CFNA standard have identical sequences.

In some embodiments, the CFNA standard comprises single-stranded genomic polynucleotides. In some embodiments, the CFNA standard comprises double-stranded genomic polynucleotides. Double-stranded genomic polynucleotides can be treated, for example by denaturation, to yield single-stranded polynucleotides if desired.

In some embodiments, the CFNA assay comprises an amplification reaction, such as digital polymerase chain reaction (dPCR), droplet digital polymerase chain reaction (ddPCR), or quantitative polymerase chain reaction (qPCR). An amplification reaction (e.g., dPCR, ddPCR, or qPCR) may be performed with amplification primers specific to the reference polynucleotide. For example, primers may be allele specific primers if a reference polynucleotide is a mutant allele.

In some embodiments, the CFNA assay comprises (a) sequencing a plurality of amplification products to generate a plurality of sequence reads, wherein the plurality of amplification products are generated by amplifying polynucleotides of the CFNA sample; and (b) analyzing the sequence reads to calculate the observed abundance of the reference polynucleotide.

In some embodiments, the CFNA standard(s) are circularized to produce a plurality of circularized genomic polynucleotides prior to amplification. In some embodiments, circularizing is effected by subjecting the CFNA standard(s) to a ligation reaction. Ligation may be accomplished, for example, by a ligase enzyme. Nucleic acids (e.g., genomic polynucleotides) having a phosphate group at a 5' terminal end and a hydroxyl group at a 3' terminal end can be circularized in the absence of a phosphate donor and/or without a preceding end repair step (e.g., using a polynucleotide kinase enzyme). In some embodiments, at least 50% of the genomic polynucleotides (e.g., at least 55%, 60%, 65%, 70%, 75%, 80%, 85%, 90%, 95% or greater than 95% of the genomic polynucleotides) comprise a phosphate group at the 5' terminal end and a hydroxyl group at the 3' end. In some embodiments, a phosphate group at a 5' terminal end and a hydroxyl group at a 3' terminal end of a genomic polynucleotide are not generated by a polynucleotide kinase, for example in an end repair process. That is, genomic polynucleotides of the CFNA standard(s) are ligatable or are ready to be ligated without the need for generating a phosphate group at a 5' terminal end and/or generating a hydroxyl group at a 3' terminal end. In some embodiments, the genomic polynucleotides of the CFNA standard(s) are ligatable or are ready to be ligated in the absence of a phosphate donor. In some embodiments, genomic polynucleotides of the CFNA standard(s) are circularized (e.g., intramolecularly ligated) with an efficiency of at least 50% (e.g., at least 55%, 60%, 65%, 70%, 75%, 80%, 85%, 90%, 95% or greater than 95%). In some embodiments, single-stranded genomic polynucleotides of the CFNA standard(s) are circularized (e.g., intramolecularly ligated) with an efficiency of at least 50% (e.g., at least 55%, 60%, 65%, 70%, 75%, 80%, 85%, 90%, 95% or greater than 95%). In some embodiments, double-stranded genomic polynucleotides of the CFNA standard(s) are circularized with an efficiency of at least 50% (e.g., at least 55%, 60%, 65%, 70%, 75%, 80%, 85%, 90%, 95% or greater than 95%). Where desired, end-repair is performed on genomic polynucleotides of the CFNA standard(s) and/or ligating genomic polynucleotides of the CFNA standard(s) is performed in the presence of a phosphate donor. In some embodiments, uncircularized polynucleotides are removed, for example by degradation or digestion, prior to amplification. In some embodiments, ligation efficiency is ascertained by a polymerase chain reaction (PCR) assay, such as a quantitative PCR assay.

Amplifying circularized genomic polynucleotides, in some embodiments, comprises isothermal amplification such as isothermal rolling circle amplification. In some embodiments, amplifying by rolling circle amplification comprises extension of random primers. Random primers having a random nucleotide sequence can hybridize and prime various random regions of a circular genomic polynucleotide. In some embodiments, amplifying comprises extension of one or more primers specific to a polynucleotide, for example that of a reference polynucleotide. In some embodiments, the primers used in primer extension may comprise at least one sequence element selected from amplification primer binding site, a sequencing primer binding site, a barcode sequence, a sample index sequence, a sequence to bind a polynucleotide to a flow cell for next generation sequence, and/or a restriction enzyme sequence. Sequence elements may facilitate further downstream analysis. For example, sequencing primer binding sequences may be useful for sequencing the amplification products.

In some embodiments, the genomic polynucleotides of the CFNA standard(s) are joined to adaptor polynucleotides to produce a plurality of adaptor-tagged genomic polynucleotides prior to amplification. In some embodiments, joining to adaptor polynucleotides is effected by subjecting the genomic polynucleotides to a ligation reaction. Ligation can be accomplished, for example, by a ligase enzyme. Nucleic acids having a phosphate group at a 5' terminal end and a hydroxyl group at a 3' terminal end can be ligated to adaptor polynucleotides in the absence of a phosphate donor and without a preceding end repair step (e.g., using a polynucleotide kinase enzyme). In some embodiments, the genomic polynucleotides are ligated to adaptor polynucleotides with an efficiency of at least 50% (e.g., at least 55%, 60%, 65%, 70%, 75%, 80%, 85%, 90%, 95% or greater than 95%). In some embodiments, single-stranded genomic polynucleotides are ligated to single-stranded adaptor polynucleotides with an efficiency of at least 50% (e.g., at least 55%, 60%, 65%, 70%, 75%, 80%, 85%, 90%, 95% or greater than 95%). In some embodiments, double-stranded genomic polynucleotides are ligated to double-stranded adaptor polynucleotides with an efficiency of at least 50% (e.g., at least 55%, 60%, 65%, 70%, 75%, 80%, 85%, 90%, 95% or greater than 95%). Where desired, end-repair is performed on the genomic polynucleotides of the CFNA standard and/or ligating the genomic polynucleotides of the CFNA standard is performed in the presence of a phosphate donor. Where desired, A-tailing (e.g., the addition of an 'A' base to the 3' end of a polynucleotide) is performed on genomic polynucleotides of a CFNA standard disclosed herein, for example prior to TA cloning. In some embodiments, the genomic polynucleotides of a CFNA standard are ligated to adaptor polynucleotides (e.g., intermolecularly ligated) after A-tailing with an efficiency of at least 50% (e.g., at least 55%, 60%, 65%, 70%, 75%, 80%, 85%, 90%, 95% or greater than 95%). In some embodiments, ligation efficiency is ascertained by a polymerase chain reaction (PCR) assay, such as a quantitative PCR assay.

An adaptor polynucleotide may contain one or more of a variety of sequence elements including, but not limited to, an amplification primer binding site, a sequencing primer binding site, a barcode sequence, a sample index sequence, a sequence to bind a polynucleotide to a flow cell for next generation sequence, and/or a restriction enzyme sequence. Amplifying the adaptor-tagged genomic polynucleotides, in some embodiments, comprises extension of one or more primers specific to a primer binding sequence of the adaptor polynucleotide. Amplification may comprise either an isothermal amplification reaction or a thermocycling amplification reaction.

In an aspect, a method, which is not claimed, of developing a cell-free nucleic acid (CFNA) assay comprises (a) performing the CFNA assay with a CFNA standard under a plurality of assay conditions to yield a set of performance metrics, wherein the CFNA standard is associated with a set of reference performance metrics when utilized in a reference CFNA assay, wherein the CFNA standard comprises a plurality of genomic polynucleotides, individual members of the plurality having a 5' terminal end and a 3' terminal end, wherein: (i) at least a subset of the plurality of genomic polynucleotides have a length ranging from about 100-300 bases; and (ii) a majority of the genomic polynucleotides have a phosphate group at a 5' terminal end and a hydroxyl group at a 3' terminal end, which majority of the genomic polynucleotides are ligatable without generating a phosphate group at a 5' terminal end and/or generating a hydroxyl group at a 3' terminal end; and (b) adjusting one or more assay conditions to improve at least one performance metric relative to at least one reference performance metric, thereby developing the CFNA assay. In some embodiments, the standard comprises at least one reference nucleic acid. The at least one reference nucleic acid may be present in the CFNA standard at an expected abundance of less than 20% (e.g., less than 10%, 5%, 1%, 0.5%, 0.1%, 0.01%, 0.001% or less than 0.001%). In some embodiments, the at least one reference nucleic acid comprises a mutant allele. In some embodiments, the mutant allele is present in the CFNA standard at an allelic frequency of about 50% (e.g., representative of a germline heterozygous mutation). The mutant allele may be present in the CFNA standard at an allelic frequency of less than 50% (e.g., less than 40%, 30%, 20%, 10%, 5%, 1%, 0.5%, 0.1%, 0.01%, 0.001% or less than 0.001%).

The development of a CFNA assay to analyze cell-free nucleic acids such as cfDNA may be challenging due to difficulty in obtaining patient-relevant material. CFNA standards disclosed herein may be representative of *in vivo* CFNA and useful in the development a CFNA assay. A CFNA assay may be performed with a CFNA standard under a plurality of assay conditions to yield a set of performance metrics. These performance metrics may include, but is not limited to, detection rate for example the proportion of reference nucleic acids that are detectable in a standard comprising a plurality of reference nucleic acids; accuracy; precision; sensitivity; specificity; reproducibility; and/or a limit of detection (e.g., detection limit). The CFNA standard when utilized in a reference assay, such as a gold standard accepted in the field, may yield a set of reference performance metrics. For example, a gold standard reference may comprise dPCR, ddPCR, and/or qPCR. The CFNA assay may be repeated while varying at least one assay condition.

In some embodiments, the assay comprises a ligation reaction. Where the assay comprises a ligation reaction, an assay condition that is varied may include ligation time or ligation temperature. For example, ligation time may be extended or shortened. For further example, ligation temperature may be increased, decreased, or cycled. Enzyme concentrations, nucleic acid template concentrations, and the relative proportions of each may also be varied to improve the assay. In some embodiments, varying an assay condition may comprise utilizing an alternative ligase enzyme or alternative ligation buffer recipe.

In some embodiments, the assay comprises an amplification reaction. Where the assay comprises an isothermal amplification reaction, an assay condition that is varied may comprise amplification time or amplification temperature. In some embodiments, the assay comprises a thermocycling amplification reaction comprising multiple cycles of various sub-stages. In a thermocycling reaction, an assay condition that is varied may include an sub-stage temperature, sub-stage length (e.g., time), or number of amplification cycles. For example, the amplification temperature of a sub-stage of a thermal cycling amplification reaction may be increased or decreased. Alternatively, the length of a sub-stage may be extended or shortened. The number of amplification cycles may also be increased or decreased. In some embodiments, enzyme concentrations, such as a polymerase enzyme, is increased or decreased. In some embodiments, an alternative polymerase or buffer recipe is used. In some embodiments, the primers of an amplification reaction may be adjusted. For example, the primer concentration may be increased, or the GC content of a primer and/or melting temperature of the primer may be varied.

By varying different assay conditions and evaluating the performance of the CFNA assay, the assay can be optimized. The assay may be optimized such that its performance metrics match or exceeds those of the gold standard.

In an aspect, systems, which are not claimed, for performing methods of the present disclosure are provided. In some embodiments, a system for estimating abundance of a target nucleic acid in a cell-free nucleic acid (CFNA) sample comprising the target nucleic acid and non-target nucleic acids comprises a quantification system configured to determine copy number of the target nucleic acid in the CFNA sample to yield an observed abundance of the target nucleic acid; a computer configured to (a) generate a calibration scheme by correlating an observed abundance of a reference nucleic acid present in a CFNA standard to an expected abundance of the reference nucleic acid present in the CFNA standard, which CFNA standard comprises a plurality of genomic polynucleotides, individual members of the plurality having a 5' terminal end and a 3' terminal end, wherein: (i) at least a subset of the plurality of genomic polynucleotides have a length ranging from about 100-300 bases; and (ii) a majority of the genomic polynucleotides have a phosphate group at a 5' terminal end and a hydroxyl group at a 3' terminal end, which majority of the genomic polynucleotides are ligatable without generating a phosphate group at a 5' terminal end and/or generating a hydroxyl group at a 3' terminal end; and (b) estimate abundance of the target nucleic acid in the CFNA sample by adjusting the observed abundance of the target nucleic acid using the calibration scheme.

A CFNA sample (e.g., cfDNA and/or cfRNA) may be obtained from a subject, for example from a biological sample of a subject. A CFNA sample may be obtained from a subject that is suspected of having a disease, pregnant, previously having a cell or tissue transplant and suspected of transplant rejection, or having a pathogenic infection. A target nucleic acid of the sample may refer to any nucleic acid that is desired to be detected. For example, in a sample obtained from a subject suspected of having a disease such as a cancer or tumor, the target nucleic acid may refer to a mutant allele known to be associated, either weakly or strongly, with the suspected cancer or tumor.

A quantification system, which is not claimed, configured to determine copy number of a target nucleic acid in a CFNA sample may implement an amplification reaction such as dPCR, ddPCR, or qPCR. Alternatively, a quantification system configured to determine copy number may (a) sequence a plurality of amplification products to generate a plurality of sequence reads, wherein the plurality of amplification products are generated by amplifying the target nucleic acid and non-target nucleic acids of the CFNA sample; and (b) analyze the sequence reads to calculate the observed abundance of the target nucleic acid.

A computer, which is not claimed, may be configured to generate a calibration scheme by correlating an observed abundance of the reference nucleic acid present in the CFNA standard to an expected abundance of the reference nucleic acid present in the CFNA standard. The computer may be configured to estimate abundance of the target nucleic acid in the CFNA sample by adjusting the observed abundance of the target nucleic acid using the calibration scheme as described elsewhere herein.

In some embodiments, the system further comprises a report generator that sends a report to a recipient. The report may contain at least one of the following: observed abundance of the target nucleic acid, estimated abundance of the target nucleic acid, observed abundance of the reference nucleic acid, expected abundance of the reference nucleic acid, and calibration scheme.

In an aspect, the present disclosure provides computer-readable media, which are not claimed, comprising code that, upon execution by one or more processors, implements a method of the present disclosure. In some embodiments, provided is a computer-readable medium comprising code that, upon execution by one or more processors, implements a method for estimating abundance of a target nucleic acid in a cell-free nucleic acid (CFNA) sample comprising the target nucleic acid and non-target nucleic acids. In some embodiments, the method comprises (a) in response to a user request, performing a quantification reaction to determine copy number of the target nucleic acid in the CFNA sample and yield an observed abundance of the target nucleic acid; (b) generating a calibration scheme by correlating an observed abundance of a reference nucleic acid present in a CFNA standard to an expected abundance of the reference nucleic acid present in the CFNA standard, which CFNA standard comprises a plurality of genomic polynucleotides, individual members of the plurality having a 5' terminal end and a 3' terminal end, wherein: (i) at least a subset of the plurality of genomic polynucleotides have a length ranging from about 100-300 bases; and (ii) a majority of the genomic polynucleotides have a phosphate group at a 5' terminal end and a hydroxyl group at a 3' terminal end, which majority of the genomic polynucleotides are ligatable without generating a phosphate group at a 5' terminal end and/or generating a hydroxyl group at a 3' terminal end; and (c) estimating abundance of the target nucleic acid in the CFNA sample by adjusting the observed abundance of the target nucleic acid using the calibration scheme.

In some embodiments, the method implemented by the code further comprises (d) generating a report that contains at least one of the following: observed abundance of the target nucleic acid, estimated abundance of the target nucleic acid, observed abundance of the reference nucleic acid, expected abundance of the reference nucleic acid, and calibration scheme.

In an aspect, the present disclosure provides kits, which are not claimed, for use in methods of the disclosure. In some embodiments, the disclosure provides a kit comprising (a) a cell-free nucleic acid (CFNA) standard comprising a plurality of genomic polynucleotides, individual members of the plurality having a 5' terminal end and a 3' terminal end, wherein: (i) at least a subset of the plurality of genomic polynucleotides have a length ranging from about 100-300 bases; and (ii) a majority of the genomic polynucleotides have a phosphate group at a 5' terminal end and a hydroxyl group at a 3' terminal end, which majority of the genomic polynucleotides are ligatable without generating a phosphate group at a 5' terminal end and/or generating a hydroxyl group at a 3' terminal end; and (b) user instructions for using the CFNA standard in a CFNA analysis.

Genomic polynucleotides of the CFNA standard, which is per se not claimed, may have a size distribution similar to that of *in vivo* cfDNA. In some embodiments, at least a subset of the plurality of genomic polynucleotides have a length of about 100-300 bases. For example, at least 30% (e.g., at least 35%, 40%, 45%, 50%, 55%, 60%, 65%, 70%, 75%, 80%, 85%, 90%, 95% or greater than 95%) of the genomic polynucleotides of the standard have a length of about 100-300 bases. In some embodiments, a majority of the genomic polynucleotides of the standard have a length of about 100-300 bases. For example, at least 50% (e.g., at least 55%, 60%, 65%, 70%, 75%, 80%, 85%, 90%, 95% or greater than 95%) of the genomic polynucleotides of the standard disclosed herein have a length of about 100-300 bases.

Genomic polynucleotides of the CFNA standard having a phosphate group at a 5' terminal end and a hydroxyl group at a 3' terminal end are ligatable or are ready to be ligated without generating a phosphate group at a 5' terminal end and/or generating a hydroxyl group at a 3' terminal end. In some embodiments, at least 50% (e.g., at least 55%, 60%, 65%, 70%, 75%, 80%, 85%, 90%, 95% or greater than 95%) of the genomic polynucleotides comprise a phosphate group at the 5' terminal end and a hydroxyl group at the 3' end. In some embodiments, the genomic polynucleotides of the CFNA standard are ligatable with an efficiency of at least 50% (e.g., at least 55%, 60%, 65%, 70%, 75%, 80%, 85%, 90%, 95% or greater than 95%). In some embodiments, the genomic polynucleotides of the CFNA standard are circularized (e.g., intramolecularly ligated) with an efficiency of at least 50% (e.g., at least 55%, 60%, 65%, 70%, 75%, 80%, 85%, 90%, 95% or greater than 95%). In some embodiments, the genomic polynucleotides of the CFNA standard are ligated to adaptor polynucleotides with an efficiency of at least 50% (e.g., at least 55%, 60%, 65%, 70%, 75%, 80%, 85%, 90%, 95% or greater than 95%). In some embodiments, ligation efficiency is ascertained by a polymerase chain reaction (PCR) assay, such as a quantitative PCR assay.

In some embodiments, the genomic polynucleotides are single-stranded polynucleotides. In some embodiments, the genomic polynucleotides are double-stranded polynucleotides. Double-stranded polynucleotides, in some cases, can be treated, for example by denaturation, to generate single-stranded polynucleotides if desired. In some embodiments, single-stranded genomic polynucleotides are circularized (e.g., intramolecularly ligated) with an efficiency of at least 50% (e.g., at least 55%, 60%, 65%, 70%, 75%, 80%, 85%, 90%, 95% or greater than 95%). In some embodiments, double-stranded genomic polynucleotides are circularized (e.g., intramolecularly ligated) with an efficiency of at least 50% (e.g., at least 55%, 60%, 65%, 70%, 75%, 80%, 85%, 90%, 95% or greater than 95%). In some embodiments, single-stranded genomic polynucleotides of the CFNA standard are ligated to single-stranded adaptor polynucleotides with an efficiency of at least 50% (e.g., at least 55%, 60%, 65%, 70%, 75%, 80%, 85%, 90%, 95% or greater than 95%). In some embodiments, double-stranded genomic polynucleotides of the CFNA standard are ligated to double-stranded adaptor polynucleotides with an efficiency of at least 50% (e.g., at least 55%, 60%, 65%, 70%, 75%, 80%, 85%, 90%, 95% or greater than 95%).

In some embodiments, a phosphate group at a 5' terminal end and a hydroxyl group at a 3' terminal end of a genomic polynucleotide of the standard are not generated by a polynucleotide kinase, for example in an end repair process. That is, genomic polynucleotides of the CFNA standard are ligatable or are ready to be ligated without generating a phosphate group at a 5' terminal end and/or generating a hydroxyl group at a 3' terminal end. In some embodiments, the genomic polynucleotides of the CFNA standard are ligatable or are ready to be ligated in the absence of a phosphate donor.

In some embodiments, the standard comprises at least one reference nucleic acid. The at least one reference nucleic acid may be present in the CFNA standard at an expected abundance of less than 20% (e.g., less than 10%, 5%, 1%, 0.5%, 0.1%, 0.01%, 0.001% or less than 0.001%). In some embodiments, the at least one reference nucleic acid comprises a mutant allele. The mutant allele may be present in the CFNA standard at an allelic frequency of about 50% (e.g., representative of a germline heterozygous mutation). The mutant allele may be present in the CFNA standard at an allelic frequency of less than 50% (e.g., less than 40%, 30%, 20%, 10%, 5%, 1%, 0.5%, 0.1%, 0.01%, 0.001% or less than 0.001%).

In some embodiments, the CFNA standard comprises multiple reference nucleic acids (e.g., at least one additional reference nucleic acid) in a single CFNA standard. In some embodiments, the expected abundance of the at least one additional reference nucleic acid present in single CFNA standard is less than 20% (e.g., less than 10%, 5%, 1%, 0.5%, 0.1%, 0.01%, 0.001% or less than 0.001%). In some embodiments, the at least one additional reference nucleic acid comprises an additional mutant allele. Where the at least one additional reference nucleic acid comprises a mutant allele, the expected abundance of the additional reference nucleic acid may be expressed as an allelic frequency. In some embodiments, the additional mutant allele is present in the single CFNA standard at an allelic frequency of about 50%. In some embodiments, the additional mutant allele is present in the single CFNA standard at an allelic frequency of less than 50% (e.g., less than 40%, 30%, 20%, 10%, 5%, 1%, 0.5%, 0.1%, 0.01%, 0.001% or less than 0.001%). In some embodiments, at least 3, 4, 5, 6, 7, 8, 9, 10 or greater than 10 reference nucleic acids are present in the single CFNA standard. In some embodiments, the kit comprises a plurality of CFNA standards (e.g., at least 2, 3, 4, 5, 6, 7, 8, 9, 10 or greater than 10 CFNA standards).

In some embodiments, the kit further comprises a ligase. A ligase may be used for intermolecular ligation (e.g., joining adaptor polynucleotides) and/or for intramolecular ligation (e.g., circularization). In some embodiments, the ligase comprises an ATP-dependent double-stranded polynucleotide ligase, NAD+ dependent DNA or RNA ligase, or single-strand polynucleotide ligase. Ligase enzymes may be wild-type, mutant isoforms, and genetically engineered variants. In some embodiments, the kit further comprises a ligation reaction buffer. A ligation reaction buffer may comprise a buffer component, small molecule ligation enhancers, and other reaction components such as ATP.

Any cell-free polynucleotide can be used by embodiments of the present disclosure. In various embodiments of the aspects herein, a cell-free nucleic acid (CFNA) sample comprises cell-free polynucleotides including, but not limited to cell-free DNA or RNA (cfDNA or cfRNA). Cell-free polynucleotides may originate from a cell but can be obtained from a non-cellular source. Cell-free polynucleotides can be derived from healthy or non-diseased cells. Cell-free polynucleotides can be derived from a diseased cell origin, such as from example a tumor or cancer cell. Cell-free polynucleotides can be of fetal origin or from a fetus. Cell-free polynucleotides can be derived from a pathogen such as a bacteria or virus, and in some cases, is indicative of a disease caused by a pathogen (e.g., bacterial infection and/or viral infection).

Cell-free polynucleotides can be obtained from a subject, such as any animal or living organism. Non-limiting examples of subjects are mammals, such as humans, non-human primates, rodents such as mice and rats, dogs, cats, pigs, sheep, rabbits and others. In some embodiments, a subject is healthy, and cell-free polynucleotides obtained from the subject may not comprise a sequence variant associated with a disease or disorder. In some embodiments, a subject is suspected of having a disease or disorder, and cell-free polynucleotides obtained from the subject may comprise a sequence variant associated with the disease or disorder. In some embodiments, a subject is pregnant, and cell-free polynucleotides obtained from the subject comprise fetal polynucleotides.

In some embodiments, a cell-free nucleic acid sample comprises a mixture of cell-free polynucleotides derived from healthy or non-diseased cells and diseased cells and/or fetal cells. In some embodiments, a cell-free nucleic acid sample obtained from a subject or patient having a disease such as a cancer may include cell-free nucleic acids from both healthy or non-diseased cells and diseased cells (e.g., cancer cells). The relative proportions of cell-free tumor nucleic acids to cell-free nucleic acids from non-diseased cells may depend on the severity of the disease or tumor. In some embodiments, cell-free tumor nucleic acids may represent less than about 10%, 9%, 8%, 7%, 6%, 5%, 4%, 3%, 2%, 1%, 0.9%, 0.8%, 0.7%, 0.6%, 0.5%, 0.4%, 0.3%, 0.2%, 0.1% or less of a nucleic acid sample. In some embodiments, a target nucleic acid of a cell-free nucleic acid sample comprises at least one sequence mutation in a gene, including a point mutation, a single nucleotide polymorphism (SNP), an insertion, a deletion, a substitution, a transposition, a translocation, a copy number variation, or another genetic mutation, alteration or sequence variation. This sequence mutation may be indicative of a disease, such as cancer. In some embodiments, the target nucleic acid comprises a chromosomal rearrangement. A chromosomal rearrangement is, for example, a deletion, duplication, inversion, or translocation.

In some embodiments, a cell-free nucleic acid sample obtained from a subject that is pregnant comprises cell-free nucleic acids from both the mother and the fetus. The relative proportions of cell-free nucleic acids from the mother and from the fetus may vary from subject to subject. In some embodiments, cell-free fetal nucleic acids may represent less than about 10%, 9%, 8%, 7%, 6%, 5%, 4%, 3%, 2%, 1%, 0.9%, 0.8%, 0.7%, 0.6%, 0.5%, 0.4%, 0.3%, 0.2%, 0.1% or less of the nucleic acid sample. In some embodiments, a target nucleic acid of a cell-free nucleic acid sample may comprise a mutant allele of the fetus, for example a mutant allele associated with a monogenic disease such as cystic fibrosis, beta-thalassemia, sickle cell anemia, spinal muscular atrophy, myotonic dystrophy, fragile-X syndrome, Duchenne muscular dystrophy and Hemophilia. In some embodiments, a target nucleic acid of a cell-free nucleic acid sample may comprise a gene or region of a chromosome that is suspected of aneuploidy or present at abnormal number (e.g., trisomy or tetrasomy).

In some embodiments, a cell-free nucleic acid sample obtained from a subject comprises a mixture of cell-free nucleic acids of the subject and cell-free nucleic acids of a pathogen, for example a bacteria or a virus. The presence of genetic material or nucleic acids from a pathogen may be useful in diagnosing and/or monitoring a pathogenic infection, such as a bacterial infection and/or viral infection. In some embodiments, cell-free nucleic acids from a pathogen such as a bacteria or virus may represent less than about 5%, 4%, 3%, 2%, 1%, 0.9%, 0.8%, 0.7%, 0.6%, 0.5%, 0.4%, 0.3%, 0.2%, 0.1% or less of a nucleic acid sample.

Cell-free polynucleotides can be obtained from various non-cellular sources. Non-limiting examples of non-cellular sources from which cell-free polynucleotides can be obtained are serum, plasma, blood, perspiration, saliva, urine, stool, semen, mucosal excretions, spinal fluid, amniotic fluid, and lymph fluid. Various methods for collecting samples of non-cellular sources from which cell-free polynucleotides can be obtained are available. In some embodiments, samples of non-cellular sources from which cell-free polynucleotides can be obtained are obtained from a subject. In some embodiments, samples are obtained by venipuncture. In some embodiments, samples are obtained by aspiration.

Various methods and commercial kits are available for obtaining cell-free polynucleotides, such as cell-free DNA, from a sample. Examples of methods and kits for extracting and isolating cell-free polynucleotides, including cell-free DNA, are phenol/chloroform extraction, phenol/chloroform/isoamyl alcohol (PCI)-glycogen extraction, NaI (sodium iodide) extraction, guanidine-resin extraction, the QIAmp DNA Blood Midi kit with carrier RNA, the ChargeSwitch serum kit, the ZR serum DNA kit, Qiagen Qubit ^{™} dsDNA HS Assay kit, Agilent^{™} DNA 1000 kit, TruSeq^{™} Sequencing Library Preparation, and the Puregene DNA purification system Blood Kit.

Cell-free polynucleotides, including cell-free DNA, can be extracted and isolated from bodily fluids through a partitioning step in which cell-free polynucleotides are separated from cells and other non-soluble components of the bodily fluid. Examples of partitioning techniques are centrifugation and filtration. In some embodiments, cells are not partitioned from cell-free polynucleotides first, but rather lysed. In some embodiments, the genomic DNA of intact cells is partitioned through selective precipitation. Cell-free polynucleotides, including DNA, may remain soluble and may be separated from insoluble genomic DNA and extracted. According to some procedures, after addition of buffers and other wash steps specific to different kits, DNA may be precipitated using isopropanol precipitation. Further clean up steps may be used such as silica based columns to remove contaminants or salts. General steps may be optimized for specific applications. Non-specific bulk carrier polynucleotides, for example, may be added throughout the reaction to optimize certain aspects of the procedure such as yield.

In some embodiments, cell-free DNA fragments are approximately uniform in length. In some embodiments, cell-free DNA fragments are not approximately uniform in length. In some embodiments, cell-free DNA fragments have an average length from about 50 to about 1000 nucleotides in length. In some embodiments, cell-free DNA fragments have an average length from about 50 to about 500 nucleotides in length. In some embodiments, cell-free DNA fragments have an average length from about 50 to about 250 nucleotides in length. In some embodiments, cell-free DNA fragments have an average length from about 50 to about 200 nucleotides in length. In some embodiments, cell-free DNA fragments have an average length from about 50 to about 100 nucleotides in length. In some embodiments, DNA fragments have an average length from about 100 to about 300 nucleotides.

The starting amount of polynucleotides in a sample may be small. In some embodiments, the amount of starting polynucleotides is less than 50 ng, such as less than 45 ng, 40 ng, 35 ng, 30 ng, 25 ng, 20 ng, 15 ng, 10 ng, 5 ng, 4 ng, 3 ng, 2 ng, 1 ng, 0.5 ng, 0.1 ng, or less. In some embodiments, the amount of starting polynucleotides is in the range of 0.1-100 ng, such as between 1-75 ng, 5 - 50 ng, or 10 - 20 ng. In general, lower starting material increases the importance of increased recovery from various processing steps. For large amounts of starting material (e.g. as purified from lab-cultured bacteria), this may not be a substantial obstacle. However, for samples where the starting material is substantially lower, recovery in this low range can be a substantial obstacle to detection of sufficiently rare variants. Accordingly, in some embodiments, sample recovery from one step to another in a method of the disclosure (e.g. the mass fraction of input into a circularization step available for input into a subsequent amplification step (or sequencing step) is about or more than about 50%, 60%, 75%, 80%, 85%, 90%, 95%, or more. Recovery from a particular step may be close to 100%. Recovery may be with respect to a particular form, such as recovery of circular polynucleotides from an input of non-circular polynucleotides.

In various embodiments of the aspects herein, the copy number of a target nucleic acid in a cell-free nucleic acid sample or a reference polynucleotide in a standard is determined to obtain an observed abundance. The copy number or number of molecules may be determined using various suitable methods including, but not limited to, digital PCR (dPCR), droplet digital (ddPCR), quantitative PCR, and next generation sequence (NGS) methods.

In some embodiments, the copy number and/or observed abundance of a target nucleic acid and/or reference polynucleotide is obtained using dPCR. dPCR refers to a technique in which a limiting dilution of the sample is made across a large number of separate PCR reactions so that most of the reactions have no template molecules and give a negative amplification result. Those reactions that are positive at the reaction endpoint are counted as individual template molecules present in the original sample in a 1 to 1 relationship. This method is an absolute counting method where solutions are partitioned into containers until there is an average probability of one molecule per two containers or when, P_{O}=(1-e^{-n/c}) = ½; where n is the number of molecules and c is the number of containers, or n/c is 0.693. Quantitative partitioning is assumed, and the dynamic range is governed by the number of containers available for stochastic separation. The molecules are then detected by PCR and the number of positive containers is counted. For example, the molecules may be detected using fluorophore-labeled, target-specific primers for PCR. Each successful amplification is counted as one molecule, independent of the actual amount of product. In various embodiments of the aspects herein, the copy number and/or observed abundance is obtained using droplet digital PCR (ddPCR). Droplet digital PCR (ddPCR) is a method for performing digital PCR that is generally based on water-oil emulsion droplet technology. ddPCR involves fractionating a sample into droplets and performing PCR amplification of the template molecules in each individual droplet. In some embodiments, multiple targets (e.g., target nucleic acids or reference polynucleotides) may be detected by multiplexing. For example, to detect two target nucleic acids, wild-type and mutant, primers specific for wild-type and primers specific for mutant may be used. Both sets of primers, wild-type and mutant, may be labeled with the same fluorophore. To distinguish between the two products, wild-type and mutant amplification products, the primers for wild-type and mutant can be used at different concentrations, for example at a 2:1 ratio. The sample can be partitioned such that each partition or droplet contains all PCR reagents, primers, and ≤1 copy of template DNA. The presence of wild-type and mutant molecules can be determined by counting the number of position amplifications in addition to comparing the fluorescent intensities, which would correlate with the primer concentration. In some cases, two different fluorophores can be used.

In some embodiments, the copy number and/or observed abundance of a target nucleic acid is obtained using "real-time PCR" or quantitative real time polymerase chain reaction (Q-PCR/qPCR/qrt-PCR) or kinetic polymerase chain reaction (KPCR). Real-time PCR or qPCR refers to a technique based on PCR used to amplify and simultaneously quantify a target DNA molecule. It enables both detection and quantification (as absolute number of copies or relative amount when normalized to DNA input or additional normalizing genes) of one or more specific sequences in a DNA sample. It is based on detection of a fluorescent signal produced proportionally during amplification of a PCR product. Generally, as the cycle proceeds, the amount of fluorescence is increased, and in response to this, a graph is formed. After a PCR standard curve is obtained from a standard sample, a PCR Ct value of an unknown sample is applied to the standard curve and quantified. Primers for qPCR amplification may be designed for any target nucleic acid, for example a particular allele of a gene.

In various embodiments of the aspects herein, target nucleic acids are detected using an amplification primer in an amplification reaction such dPCR, ddPCR, and qPCR. A primer may be of any suitable length of nucleotides or bases. For example, a primer may be about or at least about 10 bases in length (e.g., at least about 15, 20, 25, 30, 35, 40, 45, 50, 55, 60, 65, 70, 75, 80, 85, 90, 95, or about 100 bases in length). A primer may have any suitable sequence to detect a desired target, such as a sequence complementary to a mutant allele, wild-type allele, chromosomal rearrangement, particular chromosome, a reference polynucleotide, or a sequence adjacent to any of these.

In some embodiments, the copy number and/or observed abundance of a target nucleic acid is obtained using nucleic acid sequencing, such as a next-generation sequencing technique. Sequencing can be used to determine the nucleotide sequence or identity of polynucleotides in a cell-free nucleic acid sample. In some embodiments, amplification products of the polynucleotides are sequenced rather than sequencing the polynucleotides from the sample directly. Sequence data can then be analyzed to determine the copy number of the target nucleic acid in the sample.

Next-generation sequencing (NGS) techniques may allow for the determination of nucleotide sequences in a highly parallel fashion. Nucleic acid amplification and next generation sequencing (NGS) techniques include, but are not limited to, single-molecule real-time sequencing, ion semiconductor sequencing, pyrosequencing, sequencing by synthesis (SBS), sequencing by ligation (SBL), chain termination sequencing, massively parallel signature sequencing, polony sequencing, DNA nanoball sequencing, Heliscope single molecule sequencing, Nanopore DNA sequencing, sequencing by hybridization, sequencing by mass spectrometry, microfluidic Sanger sequencing, and microscopy-based sequencing techniques.

Types of templates that can be used for NGS reactions include clonally amplified templates originating from single DNA molecules and single DNA molecule templates. Methods for preparing clonally amplified templates include emulsion PCR (emPCR) and solid-phase amplification. Other methods for preparing clonally amplified templates include Multiple Displacement Amplification (MDA), wherein random hexamer primers are annealed to a template and DNA is synthesized by a high fidelity enzyme, such as typically phi29, at a constant temperature or near constant temperature.

Single-molecule templates are another type of template that can be used for NGS reactions. Spatially separated single molecule templates can be immobilized on solid supports by various methods. In one approach, individual primer molecules are covalently attached to the solid support. Adaptors are added to the templates, and the templates are then hybridized to the immobilized primers. In another approach, single molecule templates are covalently attached to the solid support by priming and extending single-stranded, single molecule templates from immobilized primers. Universal primers can then be hybridized to the templates. In yet another approach, single polymerase molecules are attached to the solid support, to which primed templates are bound.

Following template preparation, sequencing can be performed. Exemplary sequencing and imaging methods for NGS include, but are not limited to, cyclic reversible termination (CRT), sequencing by ligation (SBL), single-molecule addition (e.g., pyrosequencing), and real-time sequencing. Other sequencing methods for NGS include, but are not limited to, nanopore sequencing, sequencing by hybridization, nano-transistor array based sequencing, polony sequencing, scanning tunneling microscopy (STM) based sequencing, and nanowire-molecule sensor based sequencing. Double-ended sequencing methods can also be used for NGS.

In various embodiments of the aspects herein, the nucleic acids of a cell-free nucleic acid sample (e.g., target nucleic acid and non-target nucleic acids) or a cell-free nucleic acid standard (e.g., genomic polynucleotides of a nucleic acid standard) are circularized to produce a plurality of circularized nucleic acids and/or circularized polynucleotides prior to amplification. Circularized nucleic acids (e.g., target and non-target) may be formed from linear nucleic acids by various methods. In some embodiments, a single linear target polynucleotide is circularized by end-joining. In some embodiments, a first linear target polynucleotide is joined to a second linear target polynucleotide, and then the un-joined end of the first target polynucleotide is joined to the un-joined end of the second linear target polynucleotide to form a circular target polynucleotide comprising the first and second target polynucleotides. Nucleic acids to be circularized may be single-stranded or double-stranded. Where single-stranded circles are desired, the polynucleotide may be a single-stranded nucleic acid as originally isolated, or may be treated to render the nucleic acid single-stranded (e.g. by denaturation). In some embodiments, a method for circularizing a nucleic acid involves an enzyme, such as use of a ligase (e.g., an RNA ligase or a DNA ligase).

In some embodiments, an exonuclease step can be included to digest any unligated nucleic acids after the circularization reaction. That is, closed circles do not contain a free 5' or 3' end, and thus the introduction of a 5' or 3' exonuclease will not digest the closed circles but will digest the unligated components. After circularization, reaction products may be purified prior to amplification or sequencing to increase the relative concentration or purity of circularized polynucleotides available for participating in subsequent steps (e.g. by isolation of circular polynucleotides or removal of one or more other molecules in the reaction). For example, a circularization reaction or components thereof may be treated to remove single-stranded (non-circularized) polynucleotides, such as by treatment with an exonuclease. As a further example, a circularization reaction or portion thereof may be subjected to size exclusion chromatography, whereby small reagents are retained and discarded, or circularization products are retained and released in a separate volume. A variety of kits for cleaning up ligation reactions are available, such as kits provided by Zymo oligo purification kits made by Zymo Research. In some embodiments, purification comprises treatment to remove or degrade ligase used in the circularization reaction, and/or to purify circularized polynucleotides away from such ligase. In some embodiments, treatment to degrade ligase comprises treatment with a protease, such as proteinase K. Proteinase K treatment may follow manufacturer protocols, or standard protocols (e.g. as provided in Sambrook and Green, Molecular Cloning: A Laboratory Manual, 4th Edition (2012)). Protease treatment may also be followed by extraction and precipitation. In one example, circularized polynucleotides are purified by proteinase K (Qiagen) treatment in the presence of 0.1% SDS and 20 mM EDTA, extracted with 1:1 phenol/chloroform and chloroform, and precipitated with ethanol or isopropanol. In some embodiments, precipitation is in ethanol.

In various embodiments of the aspects herein, nucleic acids are joined to adaptor polynucleotides to produce a plurality of adaptor-tagged nucleic acids prior to amplification. Adaptor polynucleotides generally refer to oligonucleotides incorporated at the 5' and/or 3' ends of polynucleotides to facilitate one or more downstream analysis steps, for example in amplification and/or a sequencing reaction. For example, an adaptor polynucleotide may contain one or more of a variety of sequence elements including, but not limited to, an amplification primer binding site, a sequencing primer binding site, a barcode sequence, a sample index sequence, a sequence to bind a polynucleotide to a flow cell for next generation sequence, and/or a restriction enzyme sequence. Two or more sequence elements can be non-adjacent to one another (e.g. separated by one or more nucleotides), adjacent to one another, partially overlapping, or completely overlapping. For example, an amplification primer annealing sequence can also serve as a sequencing primer annealing sequence. Sequence elements can be located at or near the 3' end, at or near the 5' end, or in the interior of the adapter polynucleotide. Joining adaptor polynucleotides to nucleic acids may be effected by an enzyme, such as use of a ligase (e.g., an RNA ligase or a DNA ligase).

In some embodiments, an adaptor polynucleotide contains a universal amplification primer binding site, or one that is common to adaptor polynucleotides such that all adaptor-tagged nucleic acids can be amplified with the same primer sequence. In some embodiments, an adaptor polynucleotide is used to bind a polynucleotide to a flow cell for next generation sequencing. Non-limiting examples of next-generation sequencing methods are single-molecule real-time sequencing, ion semiconductor sequencing, pyrosequencing, sequencing by synthesis, sequencing by ligation, and chain termination. Sequencing adapters for flow cell attachment may comprise any suitable sequence compatible with next generation sequencing systems, e.g., 454 Sequencing, Ion Torrent Proton or PGM, and Illumina X10. Non-limiting examples of sequencing adapters for next generation sequencing methods include P5 and P7 adapters suitable for use with Illumina sequencing systems; TruSeq Universal Adapter; and TruSeq Indexed Adapter. In some embodiments, a sequencing adapter can be used to enrich, e.g., via amplification, such as polymerase chain reaction (PCR), for polynucleotides comprising the adapter sequence.

Non-limiting examples of enzymes that can be used to ligate a linear target polynucleotide into a circular target polynucleotide and/or join an adaptor polynucleotide to a nucleic acid include ATP-dependent double-stranded polynucleotide ligases, NAD+ dependent DNA or RNA ligases, and single-strand polynucleotide ligases. Non-limiting examples of ligases are CircLigase I and CircLigase II (Epicentre; Madison, WI), Escherichia coli DNA ligase, Thermus filiformis DNA ligase, Tth DNA ligase, Thermus scotoductus DNA ligase (I and II), T3 DNA ligase, T4 DNA ligase, T4 RNA ligase, T7 DNA ligase, Taq ligase, Ampligase (Epicentre^{®}Technologies Corp.), VanC-type ligase, 9° N DNA Ligase, Tsp DNA ligase, DNA ligase I, DNA ligase III, DNA ligase IV, Sso7-T3 DNA ligase, Sso7-T4 DNA ligase, Sso7-T7 DNA ligase, Sso7-Taq DNA ligase, Sso7-E. coli DNA ligase, Sso7-Ampligase DNA ligase, and thermostable ligases. Ligase enzymes may be wild-type, mutant isoforms, and genetically engineered variants. Ligation reactions may contain a buffer component, small molecule ligation enhancers, and other reaction components.

In some embodiments, the concentration of nucleic acids and enzyme is adjusted to facilitate intermolecular ligation rather than intramolecular ligation. In some embodiments, the reaction temperature and reaction time, or length of the reaction, is adjusted. Reaction temperatures and times can be adjusted as well. In some embodiments, 60 °C is used to facilitate intramolecular circles. In some embodiments, reaction times are between 12-16 hours. Reaction conditions may be those specified by the manufacturer of the selected enzyme. In some embodiments, joining the ends of a nucleic acid to form a circular nucleic acid (either directly to itself or to one or more other nucleic acids, e.g., a circular target nucleic acids comprises two target nucleic acids) produces a junction having a junction sequence.

In various embodiments of the aspects herein, nucleic acids, including nucleic acids of a CFNA sample and/or CFNA standard, comprise a phosphate group at a 5' terminal and end a hydroxyl group at a 3' terminal end which are not generated by a polynucleotide kinase enzyme and are ligatable (e.g., intermolecularly and intramolecularly) in the absence of a phosphate donor. Nucleic acids lacking a phosphate group at a 5' end and/or a hydroxyl group at a 3' terminal end may ligate at low efficiencies or, in some cases, may not be ligated, either intermolecularly or intramolecularly. The ends of nucleic acids lacking a phosphate group at a 5' terminal end and/or a hydroxyl group at a 3' terminal end may be repaired prior to ligation using an enzyme. End repair may include phosphorylation of a 5' terminal nucleotide, dephosphorylation of a 3' terminal nucleotide or both to generate a ligatable polynucleotide. Enzymes that can be used for end repair include polynucleotide kinases, such as T4 polynucleotide kinases, which can catalyze the transfer and exchange of Pi from the γ position of ATP to the 5'-hydroxyl terminus of polynucleotides (double-and single-stranded DNA and RNA) and nucleoside 3'-monophosphates. Any suitable phosphate donor other than adenosine triphosphate (ATP) or deoxyadenosine triphosphate (dATP) may be used for the end-repair reaction using PNK. Suitable phosphate donors include, but are not limited to, guanosine triphosphate (GTP), cytidine triphosphate (CTP), uridine triphosphate (UTP) or dexoythymine triphosphate (dTTP). Some polynucleotide kinases can also catalyze the removal of 3'-phosphoryl groups from 3'-phosphoryl polynucleotides, deoxynucleoside 3'-monophosphates and deoxynucleoside 3'-diphosphates. A combination of PNKs may also be used for the end-repair reaction. The genomic polynucleotides of the CFNA standard of the disclosure can be ligated, for example intramolecularly to generate circularized nucleic acids or intermolecularly to adaptor polynucleotides to generate adaptor tagged nucleic acids, with an efficiency of at least 50% (e.g., 55%, 56%, 60%, 65%, 70%, 75%, 80%, 85%, 90%, or 95%). The nucleic acids of the cell-free nucleic acid of the disclosure can be ligated, for example intramolecularly to generate circularized nucleic acids or intermolecularly to nucleotide adaptors to generate adaptor tagged nucleic acids, with an efficiency of at least 50% (e.g., 55%, 56%, 60%, 65%, 70%, 75%, 80%, 85%, 90%, 95% or greater than 95%) without previously being end-repaired using a polynucleotide kinase enzyme. The nucleic acids of the cell-free nucleic acid of the disclosure can be ligated, for example intramolecularly to generate circularized nucleic acids or intermolecularly to nucleotide adaptors to generate adaptor tagged nucleic acids, with an efficiency of at least 50% (e.g., 55%, 56%, 60%, 65%, 70%, 75%, 80%, 85%, 90%, 95% or greater than 95%) in the absence of a phosphate donor. In some embodiments, ligation efficiency is ascertained by a polymerase chain reaction (PCR) assay, such as a quantitative PCR assay.

In various embodiments of the aspects herein, nucleic acids (e.g., target nucleic acids, non-target nucleic acids, circularized nucleic acids, and adaptor-tagged linear nucleic acids) are amplified in primer extension and amplification reactions. Primer extension reactions can involve changes in temperature (thermocycling) or a constant temperature (isothermal). In some embodiments, primer extension reactions comprise polymerase chain reaction (PCR). PCR typically involves cycling through multiple stages of denaturation, annealing of primer pairs to opposite strands, and primer extension to exponentially increase copy numbers of the target sequence, at least some of these stages generally occurring at different reaction temperatures. Non-limiting examples of PCR amplification techniques are quantitative PCR (qPCR or realtime PCR), reverse transcription PCR (RT-PCR), digital PCR (dPCR or dePCR), target-specific PCR, and quantitative reverse transcription PCR (qRT-PCR). For example, in a conventional PCR using Taq DNA polymerase, a double stranded target nucleic acid may be denatured at a temperature >90°C, primers annealed at a temperature in the range 50-75°C, and primers extended at a temperature in the range 72-78°C. Reaction volumes typically range from a few hundred nanoliters, e.g. 200 nL, to a few hundred µL, e.g. 200 µL. Examples of polymerase enzymes that can be used for PCR are thermostable polymerases, including but not limited to, Thermus thermophilus HB8; mutant Thermus oshimai; Thermus scotoductus; Thermus thermophilus 1B21; Thermus thermophilus GK24; Thermus aquaticus polymerase (AmpliTaq^{®} FS or Taq (G46D; F667Y), Taq (G46D; F667Y; E6811), and Taq (G46D; F667Y; T664N; R660G); Pyrococcus furiosus polymerase; Thermococcus gorgonarius polymerase; Pyrococcus species GB-D polymerase; Thermococcus sp. (strain 9° N-7) polymerase; Bacillus stearothermophilus polymerase; Tsp polymerase; ThermalAce^{™} polymerase (Invitrogen); Thermus flavus polymerase; Thermus litoralis polymerase; Thermus Z05 polymerase; delta Z05 polymerase (e.g. delta Z05 Gold DNA polymerase); and mutants, variants, or derivatives thereof. Additional examples of polymerase enzymes that can be used for PCR are non-thermostable polymerases, including, but not limited to DNA polymerase I; mutant DNA polymerase I, including, but not limited to, Klenow fragment and Klenow fragment (3' to 5' exonuclease minus); T4 DNA polymerase; mutant T4 DNA polymerase; T7 DNA polymerase; mutant T7 DNA polymerase; phi29 DNA polymerase; and mutant phi29 DNA polymerase. In some embodiments, a hot start polymerase is used. A hot start polymerase is a modified form of a DNA Polymerase that requires thermal activation. Such a polymerase can be used, for example, to further increase sensitivity, specificity, and yield; and/or to further improve low copy target amplification. Typically, the hot start enzyme is provided in an inactive state. Upon thermal activation the modification or modifier is released, generating active enzyme. A number of hot start polymerases are available from various commercial sources, such as Applied Biosystems; Bio-Rad; eEnzyme LLC; Eppendorf North America; Finnzymes Oy; GeneChoice, Inc.; Invitrogen; Jena Bioscience GmbH; MIDSCI; Minerva Biolabs GmbH; New England Biolabs; Novagen; Promega; QIAGEN; Roche Applied Science; Sigma-Aldrich; Stratagene; Takara Mirus Bio; USB Corp.; Yorkshire Bioscience Ltd; and the like.

In some embodiments, primer extension and amplification reactions comprise isothermal reactions. Non-limiting examples of isothermal amplification technologies are ligase chain reaction (LCR) (e.g., U.S. Pat. Nos. 5,494,810 and 5,830,711); transcription mediated amplification (TMA) (e.g., U.S. Pat. Nos. 5,399,491, 5,888,779, 5,705,365, 5,710,029); nucleic acid sequence-based amplification (NASBA) (e.g., Malek et al., U.S. Pat. No. 5,130,238); signal mediated amplification of RNA technology (SMART) (e.g., Wharam et al., Nucleic Acids Res. 2001, 29, e54); strand displacement amplification (SDA) (e.g., U.S. Pat. No. 5,455,166); thermophilic SDA (Spargo et al., Mol Cell Probes 1996, 10:247-256; European Pat. No. 0684315); rolling circle amplification (RCA) (e.g., Lizardi, "Rolling Circle Replication Reporter Systems," U.S. Pat. No. 5,854,033); loop-mediated isothermal amplification of DNA (LAMP) (e.g., Notomi et al., "Process for Synthesizing Nucleic Acid," U.S. Pat. No. 6,410,278); helicase-dependent amplification (HDA) (e.g., U.S. Pat. Appl. US 20040058378); single primer isothermal amplification (SPIA) (e.g., WO2001020035 and U.S. Pat. No. 6,251,639); and circular helicase-dependent amplification (cHDA) (e.g., U.S. Pat. Appl. US. 10/594,095).

In some embodiments, primer extension reactions are effected by polymerases having strand-displacement activity, such as for RCA. In some embodiments, isothermal amplification comprises rolling circle amplification (RCA). A RCA reaction mixture can comprise one or more primers, a polymerase having strand displacement activity, and dNTPs. Strand displacement refers to the ability to displace down-stream DNA during synthesis. Polymerases having strand-displacement activity may have varying degrees of strand displacement activity. In some embodiments, a polymerase may have weak or no strand-displacement activity. In some embodiments, polymerases may have strong strand displacement activity. In some embodiments, polymerases with strand displacement activity may have different levels of strand-displacement activity at different reaction temperatures. In some embodiments, a polymerase may display strand displacement activity at moderate temperatures, e.g., 20°C - 37°C. In some embodiments, a polymerase may display strand displacement activity at elevated temperatures, e.g., 65°C. Reaction temperatures can be adjusted to favor a level of activity of a polymerase having strand-displacement activity. In some embodiments, a reaction temperature is at least 20 °C, 25 °C, 30 °C, 35 °C, 40 °C, 45 °C, 50 °C, 55 °C, 60 °C, 65 °C, 70 °C, 75 °C, 80 °C, 85 °C, or 90 °C. In some embodiments, a reaction temperature is between 20 °C and 80 °C. In some embodiments, a reaction temperature is between 20 °C and 70 °C. In some embodiments, a reaction temperature is between 20 °C and 60 °C. In some embodiments, a reaction temperature is between 20 °C and 50 °C. In some embodiments, various reaction temperatures can be cycled through in different stages to increase or decrease the strand displacement activity of a polymerase Non-limiting examples of polymerases having strand displacement activity include Bst DNA polymerase, large fragment; Bsu DNA polymerase, large fragment; Deep VentRTM DNA polymerase; Deep VentRTM (exo-) DNA polymerase; Klenow fragment (3' - 5' exo-); DNA polymerase I, large fragment; M-MuLV reverse transcriptase; phi29 DNA polymerase; VentR^{®} DNA polymerase; and VentR^{®} (exo-) DNA polymerase.

### EXAMPLES

The following examples are given for the purpose of illustrating various embodiments of the disclosure and are not meant to limit the present disclosure in any fashion. Changes therein and other uses which are encompassed within the invention as defined by the scope of the claims will occur to those skilled in the art.

### Example 1 - Generation of a CFNA standard

A CFNA standard disclosed herein is generated by contacting nuclei isolated from cells to an endonuclease to generate a plurality of genomic polynucleotides. Nuclei can be isolated from any population of cells, for example cells of a cell line. Prior to nuclei isolation, cells are washed with phosphate buffered saline (PBS) and pelleted by centrifugation to remove media and other reagents that may interfere with downstream processes. The cell pellet is resuspended in a cell lysis buffer containing a permeabilization agent such as Triton X-100, Tween-20, saponin, SDS, NP40, streptolysin O, proteinase K, pronase or triethanolamine. Alternatively, the cell sample is permeabilized using hypotonic shock and/or ultrasonication.

The nuclei are pelleted by centrifugation. Harvested nuclei are then treated with an enzyme such an endonuclease (e.g., caspase-activated DNase (CAD), DNase I including single-strand specific and double-strand specific DNases, DNase γ, and Endonuclease G) to cleave nucleosomal DNA into mononucleosomal-length DNA fragments (e.g., genomic polynucleotides).

The genomic polynucleotides are purified prior to use, for example by column purification or size exclusion chromatography. Genomic polynucleotides are optionally selected for size (e.g., length) prior to use, for example to enrich the proportion of genomic polynucleotides that are mononucleosomal in length.

### Example 2 - Size distribution of a CFNA standard disclosed herein

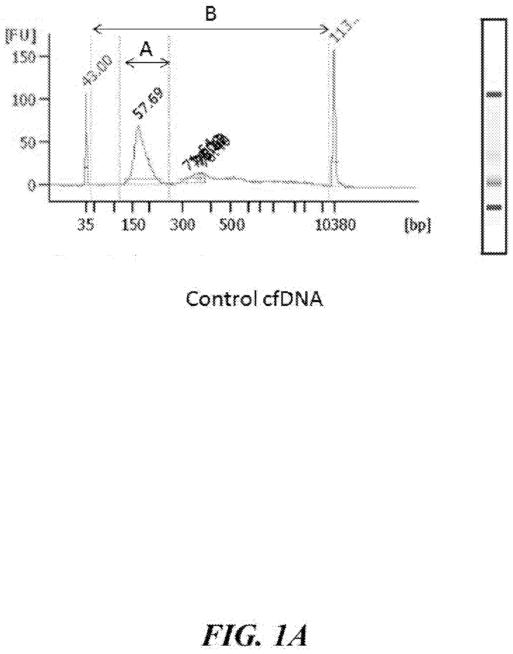

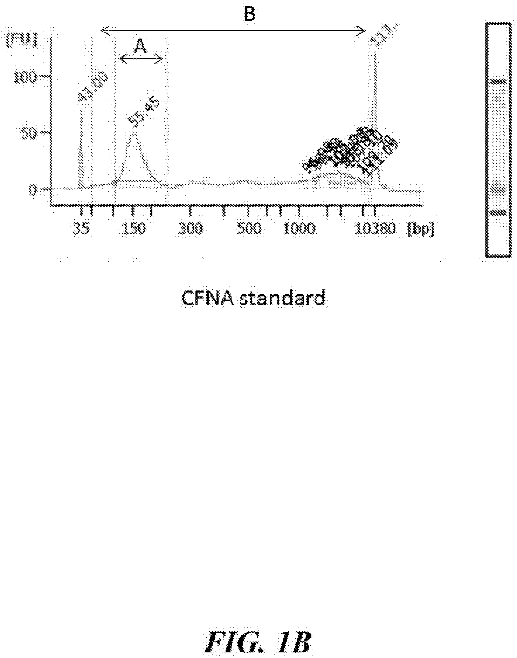

Control cfDNA and a CFNA standard as disclosed herein were analyzed by Bioanalyzer to compare the size distribution of nucleic acids in each sample. FIG. 1A shows Bioanalyzer data for the control cfDNA sample. FIG. 1B shows Bioanalyzer data for the CFNA standard generated using the methods disclosed herein. The percentage (%) of nucleic acids in each sample having a length of about 100-300 bases was quantified by taking the ratio of the area under the curve of A (nucleic acids having a length of about 100-300 bases in length) to the area under the curve of B (nucleic acids having a length of about 50-1000 bases in length). In the control cfDNA sample, about 56% of the nucleic acids have a length of about 100-300 bases. In the CFNA standard, about 50% of the nucleic acids have a length of about 100-300 bases.

### Example 3 - Comparison of inter-molecular ligation efficiency

Control cell-free DNA, a CFNA standard as disclosed herein, and sonicated genomic DNA were compared for inter-molecular ligation efficiency. Ligation reactions were carried out using a standard KAPA NGS library construction kit. Briefly, end-repair, A-tailing and adapter ligation were performed according to the manufacturer's protocol. Percentage (%) of molecules ligated were measured using a KAPA quant qPCR kit (Table 1). As shown in Table 1, the % of ligated molecules generated using the standard KAPA NGS library construction kit and sonicated genomic DNA ('Sonicated genomic DNA') is lower than with a CFNA standard disclosed herein ('CFNA standard').

**Table 1. Comparison of inter-molecular ligation efficiency**

| **Inter-molecular Ligation Efficiency** | **% of Ligated Molecules** |
|---|---|
| Control cfDNA | 61% |
| CFNA standard | 77% |
| Sonicated genomic DNA | 32% |

### Example 4 - Comparison of intra-molecular ligation efficiency

Intra-molecular ligation efficiencies of control cell-free DNA ('control'), a CFNA standard as disclosed herein ('CFNA standard'), and sonicated genomic DNA ('sonicated DNA') with an average length of ∼150 bp were compared. Ligation reactions were carried out as follows: for each ligation reaction, 30 ng of purified DNA fragments was denatured by heating at 95 °C for 30 seconds and chilled on ice for 2 minutes. Then, 8 µl of ligation mix containing 2 µl of 10x CircLigase buffer, 4 µl of 5M Betaine, 1 µl of 50mM MnCl₂, and 1 µl of CircLigase II was added to the denatured DNA samples and the reactions were incubated at 60 °C for at least 3 hours. Two replicates were setup for each sample type (control, CFNA standard, and sonicated DNA).

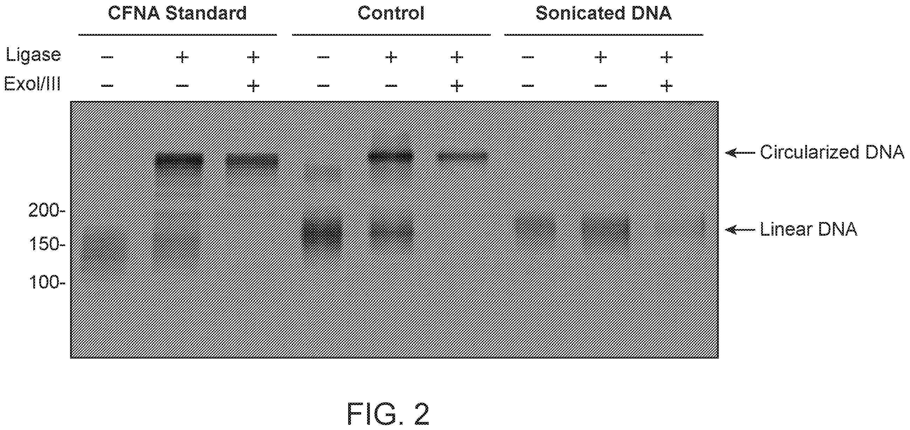

At the end the of ligation process, one of the two replicates for each sample type was treated with exonuclease mix to remove remaining linear single-stranded DNA molecules. For exonuclease treatment, ligation products were first heated at 80 °C for 45 seconds and then 1 µl of exonuclease mix (ExoI 20U/µl: ExoIII 100U/µl, at 1:2 ratio) was added. The sample was incubated on a thermal cycler at 37 °C for 30 minutes and then at 80 °C for 20 minutes. The original linear DNA, ligated DNA (e.g., ligase treated), ligated and exonuclease treated DNA samples (e.g., ligase and exonuclease treated) were then analyzed by 15% mini-PROTEAN TBE-Urea gel. As shown in FIG. 2, in both the control and CFNA standard samples, a significant portion of DNA molecules were circularized as indicated by the shifted band. However, for sonicated genomic DNA, little circularized DNA was present. The remaining linear DNA present in the lane with ligase- and exonuclease-treated sonicated DNA may be the result of incomplete exonuclease digestion.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods within the scope of these claims be covered thereby.

## Claims

1. A method for estimating abundance of a target nucleic acid present in a cell-free nucleic acid (CFNA) sample comprising the target nucleic acid and non-target nucleic acids, the method comprising:
(a) quantifying copy number of the target nucleic acid in the CFNA sample to obtain an observed abundance of the target nucleic acid, wherein the target nucleic acid and the non-target nucleic acid in the CFNA sample are subjected to a ligation reaction;
(b) generating a calibration scheme by correlating an observed abundance of a reference nucleic acid present in a CFNA standard to an expected abundance of the reference nucleic acid present in the CFNA standard, which CFNA standard comprises a plurality of genomic polynucleotides generated by contacting chromatin of nuclei isolated from cells to an endonuclease, individual members of the plurality having a 5' terminal end and a 3' terminal end, wherein:
(i) at least a subset of the plurality of genomic polynucleotides of the CFNA standard have a length ranging from about 100-300 bases;
(ii) a majority of the plurality of genomic polynucleotides of the CFNA standard have a phosphate group at a 5' terminal end and a hydroxyl group at a 3' terminal end, which majority of genomic polynucleotides are ligatable without generating a phosphate group at a 5' terminal end and/or generating a hydroxyl group at a 3' terminal end; and
(iii) the plurality of genomic polynucleotides are subjected to a ligation reaction; and
(c) estimating abundance of the target nucleic acid in the CFNA sample by adjusting the observed abundance of the target nucleic acid using the calibration scheme.

2. The method of claim 1, wherein less than 50% of individual genomic polynucleotides of the CFNA standard have identical sequences.

3. The method of claim 1, wherein at least 30% of the plurality of genomic polynucleotides have a length ranging from about 100-300 bases.

4. The method of claim 1, wherein at least 50% of the plurality of genomic polynucleotides comprise a phosphate group at the 5' terminal end and a hydroxyl group at the 3' terminal end.

5. The method of any one of claims 1-4, wherein the plurality of genomic polynucleotides of the CFNA standard are ligatable with an efficiency of at least about 50%.

6. The method of any one of claims 1-4, wherein the plurality of genomic polynucleotides of the CFNA standard are ligatable in the absence of a phosphate donor.

7. The method of claim 1, wherein the expected abundance of the reference nucleic acid present in the CFNA standard is less than 20%.

8. The method of claim 1, wherein the reference nucleic acid comprises a mutant allele.

9. The method of claim 8, wherein the mutant allele is present in the CFNA standard at an allelic frequency of less than 50%.

10. The method of claim 1, wherein generating the calibration scheme further comprises correlating an observed abundance of the reference nucleic acid present in an additional CFNA standard to an expected abundance of the reference nucleic acid present in the additional CFNA standard.

11. The method of claim 1, wherein the calibration scheme comprises a calibration algorithm, which calibration algorithm adjusts for deviation of an observed abundance of the reference nucleic acid from an expected abundance of the reference nucleic acid for a plurality of expected abundance levels of the reference nucleic acid.

12. The method of claim 1, wherein the calibration scheme comprises a line of best fit.

13. The method of claim 1, wherein the observed abundance of the target nucleic acid is determined by an amplification reaction.

14. The method of claim 1, wherein the observed abundance of the target nucleic acid present in the CFNA sample is determined by:
(a) sequencing a plurality of amplification products to generate a plurality of sequence reads, wherein the plurality of amplification products are generated by amplifying the target nucleic acid and non-target nucleic acids of the CFNA sample; and
(b) analyzing the plurality of sequence reads to calculate the observed abundance of the target nucleic acid.

15. The method of claim 14, wherein the target nucleic acid and the non-target nucleic acids are circularized to produce a plurality of circularized target nucleic acids and a plurality of circularized non-target nucleic acids prior to amplification.

## Patentansprüche

1. Verfahren zum Berechnen der Häufigkeit einer Zielnucleinsäure, die in einer zellfreien Nucleinsäure- (CFNA-) Probe enthalten ist, welche die Zielnucleinsäure und Nicht-Zielnucleinsäuren umfasst, wobei das Verfahren Folgendes umfasst:
(a) das Quantifizieren der Kopienanzahl der Zielnucleinsäure in der CFNA-Probe, um eine beobachtete Häufigkeit der Zielnucleinsäure zu erhalten, wobei die Zielnucleinsäure und die Nicht-Zielnucleinsäure in der CFNA-Probe einer Ligationsreaktion unterzogen werden;
(b) das Erzeugen eines Kalibrierungsschemas durch Korrelieren der beobachteten Häufigkeit einer in einem CFNA-Standard enthaltenen Referenznucleinsäure mit der erwarteten Häufigkeit der in einem CFNA-Standard enthaltenen Referenznucleinsäure, wobei der CFNA-Standard eine Vielzahl von Genom-Polynucleotiden umfasst, die durch Kontaktieren von Chromatin von Zellkernen, die aus Zellen isoliert wurden, mit einer Endonuclease erzeugt werden, wobei einzelne Elemente der Vielzahl ein 5'-terminales Ende und ein 3'-terminales Ende aufweisen, wobei:
(i) zumindest ein Teilsatz der Vielzahl von Genom-Polynucleotiden des CFNA-Standards eine Länge im Bereich von etwa 100 bis 300 Basen aufweist;
(ii) eine Mehrheit der Vielzahl von Genom-Polynucleotiden des CFNA-Standards eine Phosphatgruppe an einem 5'-terminalen Ende aufweisen und/oder eine Hydroxylgruppe an einem 3'-terminalen Ende aufweisen, wobei die Mehrheit von Genom-Polynucleotiden ligierbar sind, ohne eine Phosphatgruppe an einem 5'-terminalen Ende und/oder eine Hydroxylgruppe an einem 3'-terminalen Ende zu erzeugen; und
(iii) die Vielzahl von Genom-Polynucleotiden einer Ligationsreaktion unterzogen wird; und
(c) das Berechnen der Häufigkeit der Zielnucleinsäure in der CFNA-Probe durch Anpassen der beobachteten Häufigkeit der Zielnucleinsäure unter Verwendung des Kalibrierungsschemas.

2. Verfahren nach Anspruch 1, wobei weniger als 50 % von einzelnen Genom-Polynucleotiden des CFNA-Standards identische Sequenzen aufweisen.

3. Verfahren nach Anspruch 1, wobei zumindest 30 % der Vielzahl von Genom-Polynucleotiden eine Länge im Bereich von etwa 100 bis 300 Basen aufweisen.

4. Verfahren nach Anspruch 1, wobei zumindest 50 % der Vielzahl von Genom-Polynucleotiden eine Phosphatgruppe am 5'-terminalen Ende und eine Hydroxylgruppe am 3'-terminalen Ende umfassen.

5. Verfahren nach einem der Ansprüche 1 bis 4, wobei die Vielzahl von Genom-Polynucleotiden des CFNA-Standards mit einer Effizienz von zumindest etwa 50 % ligierbar ist.

6. Verfahren nach einem der Ansprüche 1 bis 4, wobei die Vielzahl von Genom-Polynucleotiden des CFNA-Standards in Abwesenheit eines Phosphat-Donors ligierbar ist.

7. Verfahren nach Anspruch 1, wobei die erwartete Häufigkeit der in dem CFNA-Standard vorhandenen Referenznucleinsäure weniger als 20 % beträgt.

8. Verfahren nach Anspruch 1, wobei die Referenznucleinsäure ein mutiertes Allel umfasst.

9. Verfahren nach Anspruch 8, wobei das mutierte Allel in dem CFNA-Standard mit einer Allel-Häufigkeit von weniger als 50 % vorkommt.

10. Verfahren nach Anspruch 1, wobei das Erzeugen des Kalibrierungsschemas weiters das Korrelierendner beobachteten Häufigkeit der in einem zusätzlichen CFNA-Standard vorhandenen Referenznucleinsäure mit der erwarteten Häufigkeit der in dem zusätzlichen CFNA-Standard vorhandenen Referenznucleinsäure umfasst.

11. Verfahren nach Anspruch 1, wobei das Kalibrierungsschema einen Kalibrierungsalgorithmus umfasst, wobei durch den Kalibrierungsalgorithmus eine Abweichung der beobachteten Häufigkeit der Referenznucleinsäure von der erwarteten Häufigkeit der Referenznucleinsäure für eine Vielzahl von erwarteten Häufigkeitswerten der Referenznucleinsäure kompensiert wird.

12. Verfahren nach Anspruch 1, wobei das Kalibrierungsschema eine Ausgleichsgerade umfasst.

13. Verfahren nach Anspruch 1, wobei die beobachtete Häufigkeit der Zielnucleinsäure durch ein Amplifikationsreaktion bestimmt wird.

14. Verfahren nach Anspruch 1, wobei die beobachtete Häufigkeit der in der CFNA-Probe vorhandenen Zielnucleinsäure wie folgt bestimmt wird:
(a) Sequenzieren einer Vielzahl von Amplifikationsprodukten, um eine Vielzahl von Sequenzauslesungen zu erzeugen, wobei die Vielzahl von Amplifikationsprodukten durch Amplifizieren der Zielnucleinsäure und von Nicht-Zielnucleinsäuren der CFNA-Probe erzeugt wird; und
(b) Analysieren der Vielzahl von Sequenzauslesungen, um die beobachtete Häufigkeit der Zielnucleinsäure zu berechnen.

15. Verfahren nach Anspruch 14, wobei die Zielnucleinsäure und die Nicht-Zielnucleinsäuren zirkularisiert werden, um vor der Amplifikation eine Vielzahl von zirkularisierten Zielnucleinsäuren und eine Vielzahl von zirkularisierten Nicht-Ziel-Nucleinsäuren zu erzeugen.

## Revendications

1. Procédé d'estimation de l'abondance d'un acide nucléique cible présent dans un échantillon d'acide nucléique acellulaire (CFNA) comprenant l'acide nucléique cible et des acides nucléiques non cibles, le procédé comprenant les étapes consistant à :
(a) quantifier un nombre de copies de l'acide nucléique cible dans l'échantillon CFNA pour obtenir une abondance observée de l'acide nucléique cible, dans lequel l'acide nucléique cible et l'acide nucléique non cible dans l'échantillon CFNA sont soumis à une réaction de ligature ;
(b) générer un schéma d'étalonnage en corrélant une abondance observée d'un acide nucléique de référence présent dans un étalon CFNA à une abondance attendue de l'acide nucléique de référence présent dans l'étalon CFNA, lequel étalon CFNA comprend une pluralité de polynucléotides génomiques générés en mettant en contact de la chromatine de noyaux isolés à partir de cellules avec une endonucléase, des membres individuels de la pluralité présentant une extrémité terminale 5' et une extrémité terminale 3', dans lequel :
(i) au moins un sous-ensemble de la pluralité de polynucléotides génomiques de la norme CFNA présente une longueur allant d'environ 100 à 300 bases ;
(ii) une majorité de la pluralité de polynucléotides génomiques de l'étalon CFNA présentent un groupe phosphate à une extrémité terminale 5' et un groupe hydroxyle à une extrémité terminale 3', laquelle majorité de polynucléotides génomiques peuvent être ligaturés sans générer de groupe phosphate à une extrémité terminale 5' et/ou générer un groupe hydroxyle à une extrémité terminale 3' ; et
(iii) la pluralité de polynucléotides génomiques sont soumis à une réaction de ligature ; et
(c) estimer l'abondance de l'acide nucléique cible dans l'échantillon CFNA en ajustant l'abondance observée de l'acide nucléique cible à l'aide du schéma d'étalonnage.

2. Procédé selon la revendication 1, dans lequel moins 50 % des polynucléotides génomiques individuels de l'étalon CFNA présentent des séquences identiques.

3. Procédé selon la revendication 1, dans lequel au moins 30 % de la pluralité de polynucléotides génomiques présentent une longueur allant d'environ 100 à 300 bases.

4. Procédé selon la revendication 1, dans lequel au moins 50 % de la pluralité de polynucléotides génomiques comprennent un groupe phosphate à l'extrémité terminale 5' et un groupe hydroxyle à l'extrémité terminale 3'.

5. Procédé selon l'une quelconque des revendications 1 à 4, dans lequel la pluralité de polynucléotides génomiques de l'étalon CFNA peuvent être ligaturés avec une efficacité d'au moins environ 50 %.

6. Procédé selon l'une quelconque des revendications 1 à 4, dans lequel la pluralité de polynucléotides génomiques de l'étalon CFNA peuvent être ligaturés en l'absence d'un donneur de phosphate.

7. Procédé selon la revendication 1, dans lequel l'abondance attendue de l'acide nucléique de référence présent dans l'étalon CFNA est inférieure à 20 %.

8. Procédé selon la revendication 1, dans lequel l'acide nucléique de référence comprend un allèle mutant.

9. Procédé selon la revendication 8, dans lequel l'allèle mutant est présent dans l'étalon CFNA à une fréquence allélique inférieure à 50 %.

10. Procédé selon la revendication 1, dans lequel la génération du schéma d'étalonnage comprend en outre la corrélation d'une abondance observée de l'acide nucléique de référence présent dans un étalon CFNA additionnel à une abondance attendue de l'acide nucléique de référence présent dans l'étalon CFNA additionnel.

11. Procédé selon la revendication 1, dans lequel le schéma d'étalonnage comprend un algorithme d'étalonnage, lequel algorithme d'étalonnage ajuste l'écart d'une abondance observée de l'acide nucléique de référence par rapport à une abondance attendue de l'acide nucléique de référence pour une pluralité de niveaux d'abondance attendus de l'acide nucléique de référence.

12. Procédé selon la revendication 1, dans lequel le schéma d'étalonnage comprend une ligne de meilleur ajustement.

13. Procédé selon la revendication 1, dans lequel l'abondance observée de l'acide nucléique cible est déterminée par une réaction d'amplification.

14. Procédé selon la revendication 1, dans lequel l'abondance observée de l'acide nucléique cible présent dans l'échantillon CFNA est déterminée par les étapes consistant à :
(a) séquencer une pluralité de produits d'amplification pour générer une pluralité de lectures de séquence, dans lequel la pluralité de produits d'amplification sont générés en amplifiant l'acide nucléique cible et les acides nucléiques non cibles de l'échantillon CFNA ; et
(b) analyser la pluralité de lectures de séquence afin de calculer l'abondance observée de l'acide nucléique cible.

15. Procédé selon la revendication 14, dans lequel l'acide nucléique cible et les acides nucléiques non cibles sont circularisés pour produire une pluralité d'acides nucléiques cibles circularisés et une pluralité d'acides nucléiques non cibles circularisés avant amplification.
